# EUROPEAN PATENT SPECIFICATION

(11) **EP 1 796 717 B1**
(45) Date of publication and mention of the grant of the patent: **29.02.2012**
(21) Application number: 05803574.2
(22) Date of filing: 07.10.2005
(51) Int. Cl.: A61K 39/12, A61K 39/116, A61K 39/00, A61K 39/295, A61K 39/05

(54) **COMBINATION VACCINE**
KOMBINATIONSIMPFSTOFF
COMPOSITION DE VACCIN COMBINE

(30) Priority: 08.10.2004 EP 04024103
(43) Date of publication of application: 20.06.2007
(73) Proprietor: Novartis Vaccines and Diagnostics GmbH, 35041 Marburg (DE)
(72) Inventor: BRÖKER, Michael, 35041 Marburg (DE)
(74) Representative: UEXKÜLL & STOLBERG
(86) International application number: PCT/EP2005/010845
(87) International publication number: WO 2006/037658

(56) References cited:
- LEIBL H ET AL: "Adjuvant/carrier activity of inactivated tick-borne encephalitis virus" VACCINE, BUTTERWORTH SCIENTIFIC. GUILDFORD, GB, vol. 16, no. 4, February 1998 (1998-02), pages 340-345, XP004099292 ISSN: 0264-410X
- OLANDER R-M ET AL: "Booster response to the tetanus and diphtheria toxoid carriers of 11-valent pneumococcal conjugate vaccine in adults and toddlers" VACCINE, BUTTERWORTH SCIENTIFIC. GUILDFORD, GB, vol. 20, no. 3-4, 12 November 2001 (2001-11-12), pages 336-341, XP004310138 ISSN: 0264-410X

## Description

The present invention relates to a combination vaccine comprising a tetanus toxoid conferring protection against tetanus, a diphtheria toxoid conferring protection against diphtheria, and an antigen from TBE-virus strain Neudörfl or TBE-virus strain K23 conferring protection against tick-borne encephalitis. The invention also relates to the use of these toxoids and antigens for the preparation of a combination vaccine for the prophylactic protection of tetanus, diphtheria and tick-borne encephalitis. In a still further aspect, the invention provides kits including these toxoids and antigens. Methods for preparing combination vaccines are also provided.

The induction of specific immunity to infectious diseases was one of the most important milestones in modern medicine. Today, a vast number of different vaccines are known in the art for the prophylactic protection of humans and animals. Among the vaccines, which are in wide use are those against tetanus and diphtheria. Tetanus and diphtheria vaccines usually contain inactivated toxins (toxoids) as antigens, which are capable of inducing protection upon administration. These toxoids are generally adsorbed on aluminium salts e.g. aluminium hydroxide, to enhance their immunogenicity.

For the matter of convenience, the tetanus and diphtheria antigens can be combined to a tetanus/diphtheria-combination vaccine which induces a protective immunity against both tetanus and diphtheria. In this way, multiple injections as well as a large total injection volume can be avoided. Several combination vaccines are already known in the art, for example, vaccines against diphtheria (D), tetanus (T) and pertussis (T; combination referred to DTP), vaccines against measles and mumps (MM) or against measles (M), mumps (M) and rubella (R; combination referred to as MMR). Further, also pentavalent vaccines such as DTP together with Hepatitis B virus (HBV) antigen and Haemophilus influenzae type B antigen (Hib) are in practical use. Further, multivalent vaccines containing antigens from acellular pertussis (aP) and inactivated polio virus (IPV) have been prepared. For example the use of the hexavalent vaccine DTaP-HBV-IPV-Hib containing antigens for conferring protection against diphtheria, tetanus, pertussis, Hepatitis B, Polio and Haemophilus influenzae type B infections was described in the prior art (Scandinavian Journal of Infectious Diseases, 2004, 36 (8), pp. 585-592).

A prerequisite for combination vaccines is a lack of "competing" antigens and a high compatibility with respect to the subject to be immunized. According to international standard applied in connection with immunization, a combined vaccine should confer a protection which is comparable to that achieved by separate vaccinations. However, the combination of antigens is a complicated process which is associated with a number of problems and uncertainties. As the individual components of a combination vaccine are not inert substances, the combination of various components into one mixture can negatively influence the immunogenicity of the individual antigenic components. For example, interactions between the different antigens or between the antigens and other components typically used in such vaccines can occur due to differences in charge, chemical residues, detergents, formaldehyde, concentration of ions etc. These changes can occur instantaneously after contacting the different antigens with each other or with other substances usually present in vaccine formulations. Moreover, these changes can also occur with a significant delay after mixing, for example during storage, shipping, etc. However, it cannot be predicted to which extent the immunogenicity of individual antigens may be influenced by mixing them to one combination. As a consequence, a situation can occur in which one or more antigens of the vaccine only confer an insufficient seroprotection against one or more of diseases which renders the product unsuitable for medical practise.

For example, Zott (Bundesgesundhbl. issue 12/1997, 498-501) reports, that replacing a whole cell pertussis component by an acellular pertussis antigen (DaPT) leads to a decrease in potency from 370 IU/dose to 84 IU/dose of the tetanus component in a combination vaccine consisting of whole cell pertussis, diphtheria toxoid and tetanus toxoid (DPT).

Similarly, a combination vaccine consisting of Haemophilus influenzae type b conjugate antigen, diphtheria and tetanus toxoid, acellular pertussis antigens and inactivated poliovirus antigens (DTaP-Hib-IPV) was shown in clinical trials to exhibit a reduced antibody formation against the Hib antigen when compared to a combination of DTaP combination given simultaneously with monovalent IPV and Hib vaccines at different sites of the body (Eskola et al., Lancet 1996; 348: 1688-1692). The consequences of such interactions leading to a loss in seroprotection can be dramatic. McVernon et al. (BMJ, 2004, 329, pp. 655-658) even blame the broad use of such a less immunogenic combination vaccines containing a Haemophilus influenzae antigen together with an a cellular pertussis component in the years 2000-2001 in the UK to be one factor of the overall increase in invasive infections with Haemophilus influenzae type B in the UK since 1998.

These results clearly demonstrate that the production of effective combination vaccines is a complex matter which depends on multidimensional interactions between the individual components of the vaccine and does not allow to extrapolate any effect of the components observed when administered separately. Thus, there is a need for combination vaccines which confer protection against several infectious diseases. This need is fulfilled by a combination vaccine according to the invention.

### Brief Summary of the invention

Against this background, the present invention provides a combination vaccine comprising a tetanus toxoid conferring protection against tetanus, a diphtheria toxoid conferring protection against diphtheria, and an antigen from TBE-virus strain Neudörfl or TBE-virus strain K23 conferring protection against tick-borne encephalitis. The vaccine according to the invention shows an excellent immunological protection. Moreover, the combination vaccine according to the present invention can be combined with certain other antigens without significantly reducing effectiveness of the vaccine composition.

### Detailed Description of the Invention

The practice of the present invention will employ, unless otherwise indicated, conventional methods of virology, microbiology, molecular biology, recombinant DNA techniques and immunology all of which are within the ordinary skill of the art. Such techniques are explained fully in the literature. See, e.g., Sambrook, et al., Molecular Cloning: A Laboratory Manual (2nd Edition, 1989); DNA Cloning: A Practical Approach, vol. I & II (D. Glover, ed.); Oligonucleotide Synthesis (N. Gait, ed., 1984); A Practical Guide to Molecular Cloning (1984); and Fundamental Virology, 2nd Edition, vol. I & II (B.N. Fields and D.M. Knipe, eds.).

It must be noted that, as used in this specification and the appended claims, the singular forms "a", "an" and "the" include plural referents unless the content clearly dictates otherwise. All scientific and technical terms used in this application have meanings commonly used in the art unless otherwise specified. As used in this application, the following words or phrases have the meanings specified.

The term "comprising" means "including" as well as "consisting" *e.g.* a composition "comprising" X may consist exclusively of X or may include something additional *e.g.* X + Y.

The term "about" in relation to a numerical value x means, for example, *x*±10%.

Diphtheria is an infectious disease which is caused by Corynebacterium diphtheriae. Certain strains of this organism produce a heat-labile polypeptide toxin having a molecular weight of approximately 62000 Da. The toxin secreted into the medium belongs to the so-called "A-B-toxins" which consist of two fragments. Fragment A (approximately 22000 Da) represents the lethal toxin which is capable to inactivate the elongation factor 2 in the target cell in the presence of NAD. Thereby, the toxin leads to an inhibition of protein synthesis and to cell death. On the other hand, fragment B (about 40000 Da) is involved in binding to the target cell and transporting fragment A through the cytoplasmic membrane into the cytoplasm, where the toxin exhibits its effect. Notably, all strains of Corynebacterium diphtheriae which produce the exotoxin comprise a specific bacteriophage (designated as beta phage) which encodes the toxin.

Diphtheria toxoid ('D') is disclosed in more detail in chapter 13 of *Vaccines,* eds. Plotkin & Orenstein, 4^{th} edition, 2004. Preferred diphtheria toxoids are those prepared by formaldehyde treatment. The diphtheria toxoid can be obtained by growing *C.diphtheriae* in growth medium (*e.g.* Fenton medium, or Linggoud & Fenton medium), which may be supplemented with bovine extract, followed by formaldehyde treatment, ultrafiltration and precipitation. The toxoided material may then be treated by a process comprising sterile filtration and/or dialysis. Quantities of diphtheria toxoid can be expressed in international units (IU). For example, the NIBSC supplies the 'Diphtheria Toxoid Adsorbed Third International Standard 1999' [Sesardic et al., 2001, Biologicals 29:107-22; NIBSC code: 98/560], which contains 160 IU per ampoule. As an alternative to the IU system, the 'Lf' unit ("flocculating units" or the "limes flocculating dose") is defined as the amount of toxoid which, when mixed with one International Unit of antitoxin, produces an optimally flocculating mixture [Module 1 of WHO's *The immunological basis for immunization series* (Galazka)]. For example, the NIBSC supplies 'Diphtheria Toxoid, Plain' [NIBSC code: 69/017], which contains 300 LF per ampoule, and also supplies 'The 1st International Reference Reagent For Diphtheria Toxoid For FJocculation Test' [NIBSC code: DIFT] which contains 900 Lf per ampoule.

Generally, vaccine booster doses approximately every 10 years are indicated for the maintenance of the acquired immunity. According to the recommendations set forth by the WHO, diphtheria vaccine is usually administered as a triple vaccine (DTP) for preschool children for primary and reinforcing ("booster") immunization while the bivalent vaccine (DT) is employed for booster doses in these preschool children. The diphtheria toxoid amount used in such vaccines is about 20 Lf/ml (Lf is the abbreviation of Limes flocculationis that is defined in detail below) or more and has a potency of at least 20 to 50 International Units/dose (IU/dose). This amount is designated as the paediatric form of the toxoid (DT).

Because such a high concentration of diphtheria antigen may induce a relative high reactogenicity in persons older than five years, diphtheria vaccines for older persons usually contain a smaller antigen amount of diphtheria toxoid. From five to seven years of age onwards, a concentration of the toxoid (Td) suitable for adults form is used (adult administration form or adult form), containing much less toxoid than the paediatric form. For example, the monovalent diphtheria vaccine for adults produced by Chiron Behring GmbH & Co KG contains 1.5 Lf diphtheria toxoid and exhibits at least 2 IU/dose.

This vaccine can be used to vaccinate persons aged five years or older and who have had a complete primary vaccination against diphtheria during their infancy and who want to boost their immune response. In many countries, a booster vaccination against diphtheria is recommended every 10 years. Because the antibody titre against tetanus also wanes over time, a booster vaccination against tetanus is also recommended every 10 years. If the recommendation to boost the tetanus and diphtheria immune response is not complied, these persons are at risk to develop diphtheria or tetanus upon infection by Corynebacterium diphtheriae or Clostridium tetani.

Tetanus is caused by the pathogenic microorganism Clostridium tetani. Microorganisms of the genus Clostridium are only capable to grow under anaerobic conditions. Clostridium tetani synthesizes several toxins, the most important of which is tetanospasmin, a heat-labile protein with a molecular weight of 150000 Da. Tetanospasmin is an extraordinary strong bacterial toxin; an amount of 10⁻⁴ µg of pure toxin is sufficient to kill a mouse within 48 hours. As for the diphtheria toxin, tetanus toxin belongs to the so-called "A-B-toxins" and consists of two sub-units, a heavy chain of about 100000 Da and a light chain of about 50000 Da. The heavy chain is responsible for binding to the target cells, whereas the light chain represents the toxic component.

The clinical symptoms of tetanus are caused by the synthesis of tetanus toxin in the infected organism. Clostridium tetani can grow in deep wound punctures that become anaerobic. Although the microorganism itself does not invade the body from the initial site of infection, the toxin produced can spread and cause severe neurological disturbance which can lead to death. Upon entry of the toxin into the central nervous system, it becomes fixed to nerve synapses by binding to one of the lipids in the membrane. Specifically, the toxin blocks the activity of the nerve factor that allows relaxation of the muscles, resulting in constant firing of motor neurons which leads to spastic paralysis typically associated with tetanus.

Tetanus toxoid ('T') is disclosed in more detail in chapter 27 of Vaccines, eds. Plotkin & Orenstein, 4th edition, 2004. Preferred tetanus toxoids are those prepared by formaldehyde treatment. The tetanus toxoid can be obtained by growing *C. tetani* in growth medium (*e.g.* a Latham medium derived from bovine casein), followed by formaldehyde treatment, ultrafiltration and precipitation. The material may then be treated by a process comprising sterile filtration and/or dialysis. Quantities of tetanus toxoid can be expressed in international units (IU). For example, the NIBSC supplies the 'Tetanus Toxoid Adsorbed Third International Standard 2000' [esardic et al., 2002, Bioogicals 30:49-68 ,NIBSC code: 98/552], which contains 469 IU per ampoule. As an alternative to the IU system, the 'Lf' unit ("flocculating units" or the "limes flocculating dose") is defined as the amount of toxoid which, when mixed with one International Unit of antitoxin, produces an optimally flocculating mixture [Module 1 of WHO's *The immunological basis for immunization series* (Galazka)]. For example, the NIBSC supplies 'The 1st International Reference Reagent for Tetanus Toxoid For Flocculation Test' [NIBSC code: TEFT] which contains 1000 Lf per ampoule.

Tick-borne encephalitis (TBE) virus is a species of the tick-borne group within the flaviviruses. The TBE-virus (also designated Central European Encephalitis virus or Russian spring-summer encephalitis virus or in German: Frühsommer-MeningoEnzephalitis-Virus (FSME-virus)) causes a variety of clinical symptoms in humans including subclinical infections, mild or severe fever and meningitis, meningoencephalitis and meningoencephalo-myelitis with serious sequelae (Kaiser, Brain 1999; 122: 2067-2078). Many regions in Europe and Asia are endemic for TBE and therefore, TBE vaccination is recommended in many European and Asian countries (Dumpis et al., Clin. Infect. Dis. 1999; 28: 882-890; Süss, Vaccine 2003; S1: 19-35).

The TBE vaccines regularly contain inactivated virus, mostly the envelope glycoprotein E, and the viral antigen - as with the T and D/d antigens - is also adsorbed to aluminium salts. The TBE vaccines available, consist of inactivated and purified viral antigens from either strain Neudörfl (Heinz et al., J. Med. Virol. 1980; 6: 213-221) or strain K23 (Klockmann et al., J. Biol. Standard. 1989; 17: 331-342). Based on serological and genetic analyses of isolates from Europe, Siberia and Far East Asia, TBE-virus has been subdivided into three closely related subtypes, but the degree of variation in the amino acid level of the glycoprotein E antigen, which confer immune protection, is low (Ecker et al., J. Gen. Virol. 1999; 80: 179-185) and therefore the TBE vaccines either based on the strain Neudörfl or K23 are interchangeable and both induce protection against a broad variety of TBE-virus strains.

The originally developed TBE vaccines may induce systemic side effects, e.g. fever, in young children and therefore TBE vaccines with a reduced amount of TBE antigen have been developed for the immunization of children (Girgsdies and Rosenkranz, Vaccine 1996; 14: 1421-1428; Zent et al., Vaccine 2003; 21: 3584-3952). For example, the TBE vaccine Encepur® for adults (Chiron Behring GmbH & Co KG) contains 1.5 µg antigen per dose, while Encepur® for children (Chiron Behring GmbH & Co KG) consists of 0.75 µg per dose and is applied to children from 1 to 11 years of age. Notably, in the case of TBE, no international calibration exist which would allow for reciting to the antigen amount in terms of its potency. Consequently, the amount of antigen used in the case of TBE is adressed in µg. Interestingly, the reactogenicity profile in young and adults persons of the TBE vaccines and the above mentioned diphtheria or tetanus/diphtheria vaccines behave quite different. While the side effects of a vaccine containing a high diphtheria antigen amount are more common in adults compared to children, the number of side reactions for the TBE vaccines is increased in children compared to adults, when the antigen amount is high.

The primary immunization against TBE consists of three vaccine doses, preferentially given at day 0, month 1-3 (after the first dose) and month 9-12 (after the second dose) for both the vaccines either based on strain Neudörfl or K23 and according to the Specific Product Characteristics of the manufacturers. Booster vaccinations are recommended every three years for persons who are at risk of TBE infection. Based on these recommendations, persons living in regions, in which the TBE-virus is endemic, may get 25 or more TBE vaccinations during their life in order to ensure protection against TBE.

Recently, a serological survey revealed, that protection against TBE upon immunisation might last for longer than three years (Rendi-Wagner et al., Vaccine 2004; 22, pp. 2743-2749) and therefore, the Vaccination Committee (Impfausschuss) of the Austrian vaccination advisory board "Oberster Sanitätsrat" in Austria recommends from 2004 onwards to expand the booster intervals for persons up to 59 years of age from three to five years upon the first booster vaccination has been carried out (Hörandl, Impfplan 2004, Welche Neuerungen gibt es? Jatros Vaccines, 1/2004, 4-5; Hörandl, FSME-Impfung. Impfschutz auf fünf Jahre verlangert. Jatros Vaccines 1/2004, 6-7). One may expect that other countries will follow these recommendations. It cannot even be excluded, that on the long term, the booster intervals for TBE vaccination may be extended to even ten years. In this case, a combination vaccine conferring protection against tetanus, diphtheria and TBE is of particular advantage, since the booster interval for the three diseases would be identical.

### Advantages of the combination vaccine of the invention

The primary immunisation against tetanus and diphtheria is generally carried out in infants aged 3 to 12 months and booster vaccinations are recommend at school entry and about primary school leave and then every 10 years. While humans become older, the efficacy of the immune system decreases and therefore, in Austria, booster immunisation against tetanus and diphtheria for persons over 60 years are recommended every 5 years in order to guarantee a sufficient immune response and a level of protective antibody titre against tetanus and diphtheria, while in Germany, the vaccination advisory board, the STIKO (Ständige Impfkommission am Robert Koch-Institut) recommends 10 years- booster intervals also for the elderly. Primary vaccination against TBE is recommended for children aged 3 years and older, adolescents and adults living in or going to TBE-virus endemic regions and booster vaccinations are recommended every three years and without any differences regarding the vaccination intervals for various age groups.

Whenever a booster vaccination against tetanus, diphtheria and TBE should be done in persons aged five years or older, two vaccine shots have to be carried out actually, namely a Td and a TBE vaccination. These two vaccines can be individually given either two to four weeks apart and concomitantly at two sites of the body, e.g. the left and the right upper arms. According to the present invention, the protection against tetanus, diphtheria and TBE can be facilitated by a combination vaccine, consisting of T, d and TBE antigens. As used herein, this combination vaccine is termed Td-TBE. Persons aged 5 to 11 years will preferably receive a Td-TBE combination vaccine with a reduced TBE antigen amount as it is already applied by the current monovalent TBE vaccines (Zent et al., Vaccine 2003; 21: 3584-3952; Ehrlich et al., Int. J. Med. Microbiol. 2004; 37: 126-127). For most of the other potential vaccinees, who are aged 12 years or older, the Td-TBE vaccine should consist of the TBE antigen amount, which is already used for immunisation of older children, adolescents and adults (Zent et al., Vaccine, 2003; 21: 738-741; Ehrlich et al., Vaccine 2003; 22: 217-223). In the context of the present invention, the adult form of the diphtheria toxoid vaccine with the reduced antigen concentration is generally designated as "d", while the paediatric form having the high amount of diphtheria toxoid used in vaccines for children is termed "D". For example, the DT-Impfstoff Behring für Kinder (a DT adsorbate vaccine for children; Chiron Behring GmbH & Co KG) contains 20 Lf tetanus toxoid with a potency of at least 40 IU/dose and 20 Lf diphtheria toxoid with a potency of at least 30 IU/dose. In comparison, the combination vaccine Td-pur® (Chiron Behring GmbH & Co KG) contains 20 Lf tetanus toxoid with a potency of at least 20 IU/dose and only 1.5 Lf diphtheria toxoid having a potency of at least 2 IU/dose.

This new combination vaccine fulfils the wish of patients, because the number of injections which are necessary to maintain a protective immune status regarding tetanus, diphtheria and TBE can be reduced significantly. In addition, this new combination vaccine supports efforts to bring the TBE vaccine away from being only a seasonal vaccination and given preferred during the spring-time in order to be protected against TBE during the following summer.

When a Td booster vaccination should be given to a person in autumn, because the interval of 10 years or more after the last Td vaccination is exceeded, subjects often refrain to get simultaneously vaccinated also against TBE due to fear of two needle injections at the same time, even when a TBE booster vaccination is indicated. In such a situation, many persons prefer to become vaccinated during springtime of the following year, but unfortunately these immunizations are then very often forgotten and the persons remain unprotected. One should have in mind, that infections by the TBE-virus can occur early in the year, e.g. the first reported TBE-virus infection in Austria in the year 2001 was as early as in February (Waldner et al., Wien. Klin. Wochenschr. 113, 454-458 (2001)). This example shows, that the delay of a TBE booster vaccination can lead to an infection, which could have been prevented by a precocious vaccination and a Td-TBE combination vaccine increases the acceptance of TBE booster vaccinations.

Whenever a Td vaccination is indicated, the use of the new Td-TBE vaccine is advisable for persons of risk for TBE infections and if the last vaccination against TBE was at least three years ago.

The value of the new Td-TBE combination vaccine can significantly reduce the number of immunisations for tetanus, diphtheria and TBE for persons living in TBE endemic areas. Assuming, that a person aged 20 years will have up to his/her 80th year of age about 7 Td booster vaccinations according to the actual German vaccination recommendations and 13 TBE booster injections according to a foreseen five-year booster interval for TBE vaccination, the combined Td-TBE booster vaccination would reduce the number of vaccinations by a total of seven injections. That means, the number of injections can be reduced by 35 %. Practically, the 20 year old person would be immunised with the Td-TBE combination vaccine by the age of 20 years to booster against tetanus, diphtheria and TBE. The next TBE booster would be given when aged 25 with a monovalent TBE vaccine. Aged 30, the person would receive again a Td-TBE booster injection, followed by a monovalent TBE vaccination when aged 30 years, etc.

In the case, that the recommendations for TBE booster vaccination will change sometime from three or five years to ten years, the management of booster immunisation against tetanus, diphtheria and TBE will be even more easier and practicable with the new Td-TBE combination vaccine and will reduce the injections by 50 %. Reduction of the number of injections not only increases the acceptance of vaccinations, but also reduces the number of side effects and therefore are an additional medicinal progress.

Even if the person to be vaccinated has not obtained a TBE vaccination so far, one can combine the Td booster immunization with the first dose of a primary TBE immunization using this new Td-TBE vaccine. The next two TBE immunizations to complete the primary TBE vaccination are then given by the monovalent TBE vaccine formulation.

The Td-TBE combination vaccine according to the invention not only supports the acceptance for TBE vaccinations, but is also a measure to enhance the general booster vaccination rates to protect against tetanus and diphtheria. There are big immunisation gaps for tetanus and diphtheria especially in the group of adults and elderly people (Hammer et al., InfFo 1/97, 35-37), such that many people have no or only a limited protection against tetanus and diphtheria. Although most of the people are generally willing and interested to become vaccinated, the reason for the low coverage rate may be seen in the fact, that patients and physicians both do not mostly think about a tetanus and/or diphtheria vaccination on the occasion of a patient's visit. If a patient has the concrete wish to become immunised against TBE, e.g. because he/she wants to travel from a TBE non-endemic to an endemic region, this is a good opportunity to vaccinate with the Td-TBE combination vaccine if indicated instead of only with the monovalent TBE vaccine.

It is generally agreed that there are no maximal intervals between vaccinations. If the recommended interval for booster vaccination against tetanus, diphtheria and/or TBE is exceeded, one single injection with this new Td-TBE vaccine is sufficient to ensure protection against these three infections.

According to the present invention, a combination vaccine is provided which comprises antigens conferring protection against at least three different infectious diseases. Specifically, the invention relates to a combination vaccine comprising at least one antigen conferring protection against tetanus, at least one antigen conferring protection against diphtheria and at least one antigen conferring protection against tick-borne encephalitis. According to a preferred embodiment of the invention, a combination vaccine is provided comprising at least one antigen from Clostridium tetani, at least one antigen from Corynebacterium diphtheriae and at least one antigen from the TBE-virus.

According to a further aspect, the invention relates to the use of at least one antigen conferring protection against tetanus, at least one antigen conferring protection against diphtheria and at least one antigen conferring protection against tick-borne encephalitis for the preparation of the combination vaccine for the prophylactic treatment of tetanus, diphtheria and tick-borne encephalitis.

The expression "Lf" as used herein is the abbreviation of the so-called Limes flocculationis. 1 Lf is defined as the amount of a toxin (or a toxoid) which leads to flocculation in the presence of 1 IU antitoxin. Thus, the Lf units indicate the content of specific protein in a sample. An amount of an antigen, such as a toxin (or a toxoid) quantity, is usually expressed as Lf or Lf/ml. The determination of the Lf value is based on the observation of Ramon (1922) according to which floccution occurs when toxin (or toxoid) and antitoxin are mixed in equivalent amounts. This means, Lf refers to the amount of antigen which precipitates in the presence of a defined amount of antibody. The Lf can be determined by methods common in the field of vaccine preparation, for example by Rocket electrophoresis (see Michael Schwanig "Diphtherie und Tetanus: Wie häufig braucht der Mensch Boosterdosen?" in: Alte und neue Impfstoffe in Deutschland. Grundlagen für künftige Entscheidungen, 27-33 (2001), Infomed Medizinische Verlagsgesellschaft mbH, Berlin).

As described above, the term Lf defines the amount of toxoid which is measured by physicochemical methods. However, the development of vaccines with antigens adsorbed to alumimum hydroxide having a strongly enhanced effectiveness over vaccines with antigens not adsorbed to aluminium hydroxide made it necessary to provide a new evaluation method for vaccines, since the binding value determined by the flocculation method does not represent a reliable value for the immunologic response. Thus, for assessment of the efficacy of a (toxoid) vaccine, the potency is much more relevant than the amount of Lf in a given vaccine (see Michael Schwanig "Diphtherie und Tetanus: Wie häufig braucht der Mensch Boosterdosen?" in: Alte und neue Impfstoffe in Deutschland. Grundlagen für künftige Entscheidungen, 27-33 (2001), Infomed Medizinische Verlagsgesellschaft mbH, Berlin). The potency of a vaccine is referred to in International Units (usually per ml (IU/ml) or per dose (IU/dose)). As used herein the "potency of a vaccine" is defined as the effectiveness of a given antigen or vaccine relative to a standard vaccine calibrated in IU. Determination of the potency is conducted according to well-known methods, for example in animal models or cell cultures. Further details can be inferred from the European Pharmacopoeia, 5^{th} edition 5.0).

The term "combination vaccine", as used herein, refers to a vaccine which confers protection against more than one infectious disease. The term embraces, for example, vaccines comprising antigens conferring protection against more than one disease caused by a pathogenic microorganism. Thus, the combination vaccine may confer protection against two, three, four, five or six different diseases. Accordingly, such vaccines are referred to as bivalent, trivalent, tetravalent, pentavalent, and hexavalent, respectively. In contrast, a vaccine which confers protection against only one specific disease is referred to as monovalent herein. Regularly, combination vaccines comprise several antigens which originate from different organisms.

According to the invention, a combination vaccine is provided which contains at least one tetanus toxoid which is suitable to induce an immunological response in a subject (such as a mammal) upon administration, wherein this response is such that the subject is protected against tetanus; at least one diphtheria toxoid which is suitable to induce an immunological response in that subject upon co-administration, wherein this response is such that the subject is protected against diphtheria; and at least one antigen from TBE virus strain Neudörfl or TBE virus strain K23 which is suitable to induce an immunological response in that subject upon administration, wherein this response is such that the subject is protected against tick-borne encephalitis. Preferably, the antigens achieve their protective effect in the immunized subject by inducing specific antibodies against these antigens (active immunity).

It has been found that the combination vaccines according to the present invention may be combined with one or more further antigens and remain effective and stable. The term "stable" as used in the context of the present invention means that the combination vaccine formulation can be kept for a period of eight days or more preferably 14 days at room temperature without any substantial loss with respect to immunogenity and stability of its distinct antigen compounds. In order to enhance stability, stabilizing agents such as saccharose can be added to the vaccine composition. Instead of saccharose or in addition thereto, other stabilisers, e.g. human serum albumin, mannose, trehalose, mannite, or polygeline can also be added as stabilizing agents. The vaccines of the present invention exhibit a pH value of preferably between 5.0 to 8.0, more preferably between 6.0 to 7.0, wherein a pH value of 6.8 to 7.8 is most preferred.

The term "effective" as used herein, refers to the fact that the antigen upon single or repeated administration to a subject (for example, a mammal) confers protection against a specific disease. As used herein the term "conferring protection" means that the antigen, upon administeration, induces an immunological response in the vaccinated subject which response is capable to protect said subject from the symptoms of subsequent infection. As used herein "immunologocal response" means that the immune system of the subject adapts to the antigen so that upon contact of the subject with the pathogenic microorganism, parts or products of that organism the symptoms associated with the disease that is caused by that organism are reduced or completely eliminated. Preferably, the immunological response induced in the vaccinated subject is based on the production of specific antibodies which are directed against the pathogenic microorganism, parts thereof, or against metabolic products produced by that microorganism, such as toxins. Preferably, the tetanus and/or diphtheria antigens induce the production of antibodies to an extent which gives rise to an geometric antibody titer with respect to that antigen of at least 0.01 IU/ml to 2.0 IU/ml, preferably 0.1 IU/ml or more and most preferably 1.0 IU/ml or more for at least 14 days after vaccination. Briefly, minimal protection is reached when the geometric antibody titer is about 0.01 IU/ml, whereas long-term protection is achieved when the titers are about 0.5 IU/ml, preferably about 1.0 IU/ml. The determination of the antibody titer of the vaccinated person can be performed by use of common methods. Preferably, the degree of protection which is reached upon administration is as high as that achieved by administering mono-, bi- or trivalent vaccines, for example a DT-combination, a TBE vaccine, or a Td-TBE combination vaccine

In the context of the present invention, the term "antigen" refers to any substance derived from a disease-causing microorganism which is upon administration suitable to confer protection against that specific disease. Particularly, the term refers to a substance originating from a pathogenic microorganism, preferably from bacteria, protozoa, viruses or fungi, which upon administration (for example, by transdermal or intramuscular injection, or oral uptake) is capable of inducing the formation of antibodies against this microorganism, parts of said microorganism or metabolic products of this organism, in a subject such as a mammal; this type of immunity is referred to as active immunity herein.

The antigen can be a killed, attenuated or inactivated viruses or a killed, attenuated or inactivated bacteria or a protein, polypeptide, peptide, glycoprotein, or a fragment thereof, a lipid, oligosaccharide, polysaccharide, lipopolysaccharide.

The antigens used in the immunogenic compositions of the present invention may be present in the composition as individual separate polypeptides. Generally, the recombinant proteins of the present invention are prepared as a GST-fusion protein and/or a His-tagged fusion protein.

However, preferably, at least two (i.e. 2, 3, 4, 5, 6, 7, 8, 9, 10, 11, 12, 13, 14, 15, 16, 17, 18, 19 or 20) of the antigens are expressed as a single polypeptide chain (a 'Hybrid' polypeptide). Hybrid polypeptides offer two principal advantages: first, a polypeptide that may be unstable or poorly expressed on its own can be assisted by adding a suitable hybrid partner that overcomes the problem; second, commercial manufacture is simplified as only one expression and purification need be employed in order to produce two polypeptides which are both antigenically useful.

Different hybrid polypeptides may be mixed together in a single formulation. Within such combinations, a antigen of the immunogenic composition of the present invention may be present in more than one hybrid polypeptide and/or as a non-hybrid polypeptide. It is preferred, however, that an antigen is present either as a hybrid or as a non-hybrid, but not as both.

Hybrid polypeptides can be represented by the formula NH₂-A-{-X-L-}*ₙ*-B-COOH, wherein: X is an amino acid sequence of an antigen of the present invention or a fragment thereof; L is an optional linker amino acid sequence; A is an optional N-terminal amino acid sequence; B is an optional C-terminal amino acid sequence; and n is 2, 3, 4, 5, 6, 7, 8, 9, 10, 11, 12, 13, 14 or 15.

If an -X- moiety has a leader peptide sequence in its wild-type form, this may be included or omitted in the hybrid antigen. In some embodiments, the leader peptides will be deleted except for that of the -X- moiety located at the N-terminus of the hybrid protein i.e. the leader peptide of X₁ will be retained, but the leader peptides of X₂ ... Xₙ will be omitted. This is equivalent to deleting all leader peptides and using the leader peptide of X₁ as moiety -A-.

For each n instances of (-X-L-), linker amino acid sequence - L- may be present or absent. For instance, when *n*=2 the hybrid may be NH₂-X₁-L₁-X₂-L₂-COOH, NH₂-X₁-X₂-COOH, NH₂-X₁-L₁-X₂-COOH, NH₂-X₁-X₂-L₂-COOH, *etc*. Linker amino acid sequence (s) -L- will typically be short, *e.g.*,20 or fewer amino acids (*i.e.*, 20, 19, 18, 17, 16, 15, 14, 13, 12, 11, 10, 9, 8, 7, 6, 5, 4, 3, 2, 1). Examples include short peptide sequences which facilitate cloning, poly-glycine linkers (Gly*ₙ* where *n* = 2, 3, 4, 5, 6, 7, 8, 9, 10 or more), and histidine tags (His*ₙ* where *n* = 3, 4, 5, 6, 7, 8, 9, 10 or more). Other suitable linker amino acid sequences will be apparent to those skilled in the art. A useful linker is GSGGGG, with the Gly-Ser dipeptide being formed from a *Bam*HI restriction site, which aids cloning and manipulation, and the (Gly)₄ tetrapeptide being a typical poly-glycine linker.

-A- is an optional N-terminal amino acid sequence. This will typically be short, e.g., 40 or fewer amino acids (*i.e.*, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking or short peptide sequences which facilitate cloning or purification (*e.g.*, a histidine tag His*ₙ* where *n* = 3, 4, 5, 6, 7, 8, 9, 10 or more). Other suitable N-terminal amino acid sequences will be apparent to those skilled in the art. If X₁ lacks its own N-terminus methionine, -A- is preferably an oligopeptide (*e.g.*, with 1, 2, 3, 4, 5, 6, 7 or 8 amino acids) which provides a N-terminus methionine.

-B- is an optional C-terminal amino acid sequence. This will typically be short, e.g., 40 or fewer amino acids (i.e., 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning or purification (*e.g.*, His*ₙ* where *n* = 3, 4, 5, 6, 7, 8, 9, 10 or more), or sequences which enhance protein stability. Other suitable C-terminal amino acid sequences will be apparent to those skilled in the art.

The individual antigens of the immunogenic composition within the hybrid (individual -X- moieties) may be from one or more strains or from one or more M types. Where *n*=2, for instance, X₂ may be from the same strain or type as X₁ or from a different strain or type. Where n=3, the strains might be (i) X₁=X₂=X₃, (ii). X₁=X₂≠X₃, (iii) X₁≠X₂=X₃, (iv) X₁≠X₂≠X₃, or (v) X₁=X₃≠X₂, etc.

Where hybrid polypeptides are used, the individual antigens within the hybrid (*i.e.* individual -X- moieties) may be from one or more strains. Where n=2, for instance, X₂ may be from the same strain as X₁ or from a different strain. Where n=3, the strains might be (i) X₁=X₂=X₃ (ii) X₁=X₂≠X₃ (iii) X₁≠X₂=X₃ (iv) X₁≠X₂≠X₃ or (v) X₁=X₃≠X₂, etc. By way of example, a TBE virus antigen selected from one or more epidemiologically prevalent serotypes set out in Table 1 of Ecker et al (1999) J General Virology 80: 179-185.

It will be appreciated that also fragments, derivatives and other modified forms of such molecules may be used as antigens.

The invention also provides nucleic acid encoding polypeptides of the invention. Furthermore, the invention provides nucleic acid which can hybridise to this nucleic acid, preferably under "high stringency" conditions (*e.g.* 65°C in a 0.1xSSC, 0.5% SDS solution).

Polypeptides of the invention can be prepared by various means (*e.g.* recombinant expression, purification from cell culture, chemical synthesis, *etc*.) and in various forms (*e.g.* native, fusions, non-glycosylated, lipidated, etc.). They are preferably prepared in substantially pure form (*i.e.* substantially free from other host cell or non host cell proteins).

Nucleic acid according to the invention can be prepared in many ways (*e.g.* by chemical synthesis, from genomic or cDNA libraries, from the organism itself, *etc*.) and can take various forms (*e.g.* single stranded, double stranded, vectors, probes, *etc.*). They are preferably prepared in substantially pure form (*i.e.* substantially free from other host cell or non host cell nucleic acids).

The term "nucleic acid" includes DNA and RNA, and also their analogues, such as those containing modified backbones (*e.g.* phosphorothioates. *etc*.), and also peptide nucleic acids (PNA), *etc*. The invention includes nucleic acid comprising sequences complementary to those described above (*e.g.* for antisense or probing purposes).

Moreover, the antigen can be a nucleic acid, such as a deoxynucleic acid or a ribonucleic acid. Vaccines based on DNA or RNA have attracted much interest in the recent years. In this approach, the nucleic acid comprised by the vaccine is translated to the respective protein or polypeptide in the subject's body after administration. In the case of DNA or RNA vaccines, it is particularly advantageous that antigenicity of the nucleic acid can be modified by simple sequence modifications.

The invention also provides a process for producing a polypeptide of the invention, comprising the step of culturing a host cell transformed with nucleic acid of the invention under conditions which induce polypeptide expression. By way of example, the glycoprotein E of the TBE virus may be expressed by recombinant technology and used to develop an immunogenic composition comprising a recombinant subunit TBE vaccine. Alternatively the viral capsid protein gene may also be used as a target for a TBE combination vaccine.

The heterologous host may be prokaryotic (e.g. a bacterium) or eukaryotic. It is preferably E.coli, but other suitable hosts include *Bacillus subtilis, Vibrio cholerae, Salmonella typhi, Salmonella typhimurium, Neisseria lactamica, Neisseria cinerea, Mycobacteria (e.g. M.tuberculosis),* yeasts, etc.

The polypeptides of the invention can be prepared in various forms (e. g. native, fusions, glycosylated, non-glycosylated etc.).

Polypeptides of the invention may be attached to a solid support.

Polypeptides of the invention may comprise a detectable label (e. g. a radioactive or fluorescent label, or a biotin label).

The invention also provides a process for producing a polypeptide of the invention, comprising the step of synthesising at least part of the polypeptide by chemical means.

Further, the invention provides a process for producing nucleic acid of the invention, wherein the nucleic acid is prepared (at least in part) by chemical synthesis.

The invention also provides a process for producing nucleic acid of the invention, comprising the step of amplifying nucleic acid using a primer-based amplification method (*e.g.* PCR).

The invention further provides a process for producing a protein complex of the invention, comprising the step of contracting a class I MHC protein with a polypeptide of the invention, or a fragment thereof.

The invention provides a process for producing a protein complex of the invention, comprising the step of administering a polypeptide of the invention, or a fragment thereof, to a subject. The process may comprise the further step of purifying the complex from the subject.

The invention also provides a process for producing a composition comprising admixing a polypeptide and/or a nucleic acid of the invention with a pharmaceutically acceptable carrier or diluent.

The antigens may be obtained in pure form from different manufacturers, or may be prepared by purifying the respective antigenic molecules from a suitable source. If the antigen is, for example, a bacterial exopolypeptide which is secreted by the producing organism, it may be readily purified from culture supernatants by common chromatographic methods (for example, the diphtheria toxin). Alternatively, if the antigen is a nucleic acid, it may be obtained by methods involving polymerase-chain-reaction etc. If the antigen is a whole virus fraction, it may be purified, for example, from a cell culture supernatant after infection and lysis of the cells by use of seperators and filters. Further examples of antigens and means for their isolation and purification methods have been described by von Behring et al., Sera und Impfstoffe. In: Ullmanns Encyklopädie der technischen Chemie. Band 21, 273-310 (1982), Verlag Chemie GmbH, Weinheim. Alternatively, peptide, polypeptide or protein antigens can also be produced by recombinant DNA-techniques, such as heterologous expression. Methods suitable for such production processes are known in the art.

It will be appreciated by those skilled in the art that antigenic molecules such as peptides, polypeptides or proteins may also be produced by recombinant DNA-techniques, such as heterologous expression. Methods suitable for such production processes are well known in the art and comprise, for examples, the baculovirus expression system, the yeast-two-hybrid system, chinese-hamster-ovary (CHO) system and many others.

The term "antigen" in particular embraces exoproteins or exopolypeptides which are secreted by microorganisms, such as bacteria. Also embraced are endoproteins or endopolypeptides which are released from the producing cell upon lysis. Such exoproteins/exopolypeptides or endoproteins/endopolypeptides can be, for example, protein toxins or peptide toxins. In a vaccine, these toxins are preferably used in a modified form, because in many cases the pure toxin is not suitable for direct injection. Here, the toxins are usually modified chemically so that they retain their antigenicity but loose (part of) their toxicity. Such modified toxins are usually referred to as toxoids. The formation of toxoids may be achieved, for example, by treating a toxin with formaldehyde which blocks some of the free amino groups in the molecule, thereby converting the toxin to a toxoid. Preferably, the toxoid is much less effective compared to the corresponding toxin and therefore can be given safely and in high doses. Examples of toxoid antigens are the diphtheria toxoid and the tetanus toxoid. Methods of producing toxoids are well known in the art and are summarized, for example, in described by von Behring et al., see above. It will be appreciated that also fragments of the polypeptide toxins or toxoids, in unmodified or modified form, may be used in practising the present invention.

Where immunization against whole microorganisms is aimed at, the antigen according to the invention can be a part of the microorganism, such as a specific protein of the outer membrane of gramnegative bacteria, or the microorganism itself. In the latter case, the microorganism can be killed by agents such as formaldehyde, phenol or heat, and the dead cells can be subsequently incorporated as an antigen into a vaccine and injected. In these cases, the antigen consists of a whole cell/virus fraction or of an extract of such a fraction. This mode of vaccination is, for example, regularly applied with pathogenic organisms such as Vibrio cholerae, Bordetella pertussis and Yersinia pestis.

The at least one antigen conferring protection against tetanus in the combination vaccine is a tetanus toxoid, and the at least one antigen conferring protection against diphtheria in the combination vaccine is a diphtheria toxoid. This means, that the combination vaccine according to the present invention may, next to other antigenic substances, may comprise toxoids which can be obtained from toxin-producing strains of Clostridium tetani and Corynebacterium diphtheriae.

Generally, toxoids can be prepared from toxins in cultures of Clostridium tetani and Corynebacterium diphtheriae. The toxins produced by these organisms have been described with respect to their primary structure; see for example Eisel, U et al., EMBO J. 1986 Oct; 5(10):2495-502.

The toxoids of Corynebacterium diphtheriae and Clostridium tetani can also be purchased in the form of a DT combination vaccine (DT-Impfstoff Behring für Kinder; Diphtherie-Tetanus-Adsorbat-Impfstoff für Kinder (Chiron Behring GmbH & Co KG) which contains 20 Lf tetanus toxoid with a potency of at least 40 IU/dose and 20 Lf diphtheria toxoid with a potency of at least 30 IU/dose. Additionaly, the combination vaccine Td-pur® (Chiron Behring GmbH & Co KG) contains 20 Lf tetanus toxoid with a potency of at least 20 IU/dose and only 1.5 Lf diphtheria toxoid having a potency of at least 2 IU/dose. According to the invention, these vaccines or the antigens which are components of these vaccines, can be directly used as starting materials for preparing the combination vaccines of the invention. Alternatively, as mentioned above, the toxoids can also be prepared by purification of the toxins from the supernatant of cultures of the respective organisms. Subsequently, the purified toxins can be modified according to methods known in the art in order to produce toxoids.

According to the invention, the at least one tetanus toxoid may be present in an amount which corresponds to a potency of about 1-70 IU/dose. When used in a combination vaccine for children under 12 years of age, the antigen may be present in an amount which corresponds to a potency of about 20-60 IU/dose, whereas a potency of about 30-50 IU/dose is preferred, and a potency of at least 40 IU/dose is most preferred. When used in a combination vaccine for children of at least 12 years of age and adults the antigen may be present in an amount which corresponds to a potency of about 5-40 IU/dose, whereas a potency of about 10-30 IU/dose is preferred, and a potency of at least 20 IU/dose is most preferred.

Similarly, the at least one diphtheria toxoid may be present in an amount which corresponds to a potency of 0.1-70 IU/dose. When used in a combination vaccine for children under 12 years of age, the antigen may be present in an amount which corresponds to a potency of about 10-50 IU/dose, whereas a potency of about 20-40 IU/dose is preferred, and a potency of at least 30 IU/dose is most preferred. When used in a combination vaccine for children of at least 12 years of age and adults the antigen may be present in an amount which corresponds to a potency of about 0.5-10 IU/dose, whereas a potency of about 0.75-5 IU/dose is preferred, and a potency of at least 2 IU/dose is most preferred.

According to a preferred embodiment of the invention, the at least one toxoid in the combination vaccine conferring protection against tetanus is derived from Clostridium tetani strain Massachusetts F1. The strain can be obtained from Commonwealth of Massachusetts, Departure of Publich Health, Division Biologic Laboratories, Boston. Furthermore, the at least one toxoid conferring protection against diphtheria is preferably derived from Corynebacterium diphtheriae strain Massachusetts 8 Park Williams (see J.H. Mueller, 1939, J. Immunol. 37, 103-111; Russell, L., and R. Holmes, 1985, Infect. Immun. 47:575-578). This strain can be obtained from Massachusetts Antitoxin and Vaccine Laboratory, Forest Hills, Boston.

In the case of virus vaccines, the antigen of the vaccine can consist of a whole virus fraction or an extract of a whole virus fraction as well as of distinct pure molecules derived from the virus such as DNA, RNA, proteins, peptides or fragments of proteins and peptides. As used herein, the expression "whole virus fraction" means that the virus, which is isolated for the vaccine formulation is not treated such that a subunit antigen fraction is prepared. In contrast, the complete fraction contains the virus obtained from a culture after separating from the medium components. The complete fraction can also be used as an antigen. Also embraced by the term are fractions which are enriched with respect to a certain antigen. For example, in the case of the TBE-virus, a fraction is used which is enriched with respect to glycoprotin E; however the fraction also contains other virus components, i.e. it is not a fraction containing purified virus. Processes and means for obtaining a "whole virus fraction" are described, for example, in "Vaccines", Plotkin and Orenstein (eds.), 2004.

The at least one antigen conferring protection against tick-borne encephalitis is characterized in that it originates from TBE-flavivirus strain Neudörfl or TBE-flavivirus strain K23, both of which are known with respect to their sequence in the prior art (Heinz et al., J. Med. Virol. 1980; 6: 213-221; Klockmann et al., J. Biol. Standard. 1989; 17: 331-342).

According to a preferred embodiment of the invention, the at least one antigen conferring protection against tick-borne encephalitis derives from a TBE-flavivirus, i.e. it is an antigen of a TBE-virus. According to a particularly preferred embodiment of the invention, the antigen from a TBE-flavivirus is the envelope glycoprotein E or fragments thereof. The primary structure of this glycoprotein is known in the prior art and it is described for example in the publication of Ecker et al., J. Gen. Virol. 80, 179-185 (1999). According to an alternative embodiment of the invention, the antigen of the TBE-virus is a whole virus fraction.

According to the present invention, the amount of said at least one antigen conferring protection against tick-borne encephalitis, such as the antigen derived from a TBE-virus, may range in the combination vaccine from about 0.01 µg/dose to about 10 µg/dose, if a whole virus vaccine such as Encepur® is used. Alternatively, in case pure components of the virus are used, the amount depends on the nature of the antigen molecule. If, for example, purified glycoprotein E should be incorporated into the combination vaccine, an amount of about 0.1 to 5 µg/dose, preferentially 0.5 to 2.5 µg/dose may be used. Preferably, the antigen conferring protection against tick-borne encephalitis is present in an amount of 1.5 µg/dose.

According to a preferred aspect, the invention provides a combination vaccine, wherein the at least one antigen conferring protection against tetanus is present in an amount which corresponds to a potency of at least 20 IU/dose, the at least one antigen conferring protection against diphtheria is present in an amount which corresponds to a potency of at least 2 IU/dose, and the at least one antigen conferring protection against tick-borne encephalitis is present in an amount of at least 0.75 µg/dose.

As used herein, the term "dose" refers the amount of a medicament or vaccine which is to be administered. Specifically, the term refers to a dosage unit form of a medicament or vaccine. For example, if the medicament in question is a liquid, the dose will be referred to in terms of the volume. Suitable doses in the context of the present invention have a volume of 0.1-2.0 ml, preferably 0.5-1.0 ml. If the medicament in question is present as a pill, the dose will be referred to in terms of the number of pills which is to be administered. In the context of the present invention, the term dose is preferably used to address a volume which is to be administered. As used herein, the potency of the combination vaccine according to the invention is referred to as IU/dose. This means that the potency as expressed by the specific IU value applies if the dose is administered. For example, if a vaccine exhibits a potency of 20 IU/dose and the dose is present in a volume of 1 ml, an effect of protection is achieved which corresponds to 20 IU (relative to the respective calibration vaccine) when the complete 1 ml is administered.

The combination vaccine according to the invention can further comprise at least one additional antigen conferring protection against a further disease, for example, an antigen from another pathogenic microorganism, such as a virus and/or a bacterial pathogen, so that tetravalent, pentavalent or hexavalent vaccines can be produced. Preferably, the one or more additional antigen is capable of conferring protection against a disease or medical condition selected from a group of pertussis, polio, hepatitis A, meningococcal diseases, Lyme disease. Examples for such multivalent vaccines which can be prepared in accordance with the present invention comprise (but are not restricted to) a Td-aP-TBE vaccine, a Td-IPV-TBE vaccine, and a Td-aP-IPV-TBE vaccine.

Generally the optimum amount of a specific antigen can be evaluated using standard methods involving measuring the antibody titers as well as other responses of the vaccinated subject. The vaccination course can be adopted to the proposal of national or international authorities. It is expected that the antigens of the combination vaccine according to the invention are used in an amount which produces immunological responses which confer protection. Specifically, these amounts produce geometric mean titers of antibodies against the specific antigen (tetanus or diphtheria) of approximately at least 0.01 IU/ml preferably at least 0.1 IU/ml and most preferably at least 1.0 IU/ml for at least 14 days after vaccination.

According to a preferred embodiment of the present invention, the antigens of Clostridium tetani, Corynebacterium diphtheriae, and the TBE-virus are present in the vaccine absorbed to an adjuvant. Adjuvants are frequently used in the formulation of vaccine compositions. In the context of the present invention, the term "adjuvant" refers to any substance which leads to an enhanced immunological reaction or response of the subject which receives the immunization as compared to administering the vaccine without the adjuvant. Such an enhanced response can be observed, for example, as an enhanced antibody titer against the respective antigen.

Adjuvants for use with the invention include, but are not limited to, one or more of the following: mineral containing compositions, such as aluminum salts and/or calcium salts; oil-emulsions, such as squalene-water emulsions, for example MF59 (5% Squalene, 0.5% Tween 80, and 0.5% Span 85; Saponin formulations including QS7, QS17, QS18, QS21, QH-A, QH-B and QH-C; immunostimulatory oligonucleotides, such as sequences containing a CpG motif; bioadhesives and mucoadhesives, such as esterified hyaluronic acid microspheres (Singh et al. (2001) J. Cont. Rele. 70:267-276) or mucoadhesives such as cross-linked derivatives of poly(acrylic acid), polyvinyl alcohol, polyvinyl pyrollidone, polysaccharides and carboxymethylcellulose; biodegradable microparticles such as particles of a poly-α-hydroxy acid, a polyhydroxybutyric acid, a polyorthoester, a polyanhydride, a polycaprolactone, etc.; liposomes; polyoxyethylene ethers and polyoxyethylene esters (see for example WO99/52549), polyphosphazene (PCPP) formulations; muramyl peptides; imidazoquinolone compounds; human immunomodulators such as interleukins (*e.g.* IL-1, IL-2, IL-4, IL-5, IL-6, IL-7, IL-12, *etc.*), interferons (*e.g.* interferon-γ), macrophage colony stimulating factor, and tumor necrosis factor; and others.

Mineral containing compositions which include mineral salts, such as aluminum salts and/or calcium salts are particularly preferred in the present invention as adjuvants. The invention includes mineral salts such as hydroxides (*e.g.* oxyhydroxides), phosphates (*e.g.* hydroxyphosphates, orthophosphates), sulfates, *etc*. (*e.g.* see chapters 8 & 9 of Vaccine Design (1995) eds. Powell & Newman. ISBN: 030644867X. Plenum.), or mixtures of different mineral compounds (*e.g.* a mixture of a phosphate and a hydroxide adjuvant, optionally with an excess of the phosphate), with the compounds taking any suitable form (*e.g.* gel, crystalline, amorphous, *etc*.), and with adsorption to the salt(s) being preferred. The mineral containing compositions may also be formulated as a particle of metal salt (see, for example, WO 00/23105). According to a preferred embodiment of the present invention, the adjuvant used is an aluminium salt. According to a particular preferred embodiment of the present invention, the aluminium salt used is aluminium hydroxide. The precise amount of adjuvant to be used depend on its nature. For example, if aluminium hydroxide is the adjuvant, it can be used in an amount of 2-20 g/l antigen suspension.

As used herein, the term "combination vaccine" refers to an immunogenic composition comprising, for example polypeptide antigens or nucleic acid molecules.

The pH of such compositions preferably is between 6 and 8, preferably about 7. The pH can be maintained by the use of a buffer. The composition can be sterile and/or pyrogen-free. The composition can be isotonic with respect to humans. Vaccines according to the invention may be used either prophylactically or therapeutically, but will typically be prophylactic and can be used to treat animals (including companion and laboratory mammals), particularly humans.

Compositions of the invention may be administered in conjunction with one or more antigens for use in therapeutic, prophylactic, or diagnostic methods of the present invention. Preferred antigens include those listed below. Additionally, the compositions of the present invention may be used to treat or prevent infections caused by any of the below-listed pathogens. In addition to combination with the antigens described below, the compositions of the invention may also be combined with an adjuvant as described herein.

Antigens for use with the invention include, but are not limited to, one or more of the following antigens set forth below, or antigens derived from one or more of the pathogens set forth below:

### A. Bacterial Antigens

Bacterial antigens suitable for use in the invention include proteins, polysaccharides, lipopolysaccharides, and outer membrane vesicles which may be isolated, purified or derived from a bacteria. In addition, bacterial antigens may include bacterial lysates and inactivated bacteria formulations. Bacteria antigens may be produced by recombinant expression. Bacterial antigens preferably include epitopes which are exposed on the surface of the bacteria during at least one stage of its life cycle. Bacterial antigens are preferably conserved across multiple serotypes. Bacterial antigens include antigens derived from one or more of the bacteria set forth below as well as the specific antigens examples identified below. *Neisseria meningitides: Meningitides* antigens may include proteins (such as those identified in References 1 - 7), saccharides (including a polysaccharide, oligosaccharide or lipopolysaccharide), or outer-membrane vesicles (References 8, 9, 10, 11) purified or derived from *N*. *meningitides* serogroup such as A, C, W135, Y, and/or B. Meningitides protein antigens may be selected from adhesions, autotransporters, toxins, Fe acquisition proteins, and membrane associated proteins (preferably integral outer membrane protein).

*Streptococcus pneumoniae: Streptococcus pneumoniae* antigens may include a saccharide (including a polysaccharide or an oligosaccharide) and/or protein from *Streptococcus pneumoniae.* Saccharide antigens may be selected from serotypes 1, 2, 3, 4, 5, 6B, 7F, 8, 9N, 9V, 10A, 11A, 12F, 14, 15B, 17F, 18C, 19A, 19F, 20, 22F, 23F, and 33F. Protein antigens may be selected from a protein identified in WO 98/18931, WO 98/18930, US Patent No. 6,699,703, US Patent No. 6,800,744, WO 97/43303, and WO 97/37026. Streptococcus pneumoniae proteins may be selected from the Poly Histidine Triad family (PhtX), the Choline Binding Protein family (CbpX), CbpX truncates, LytX family, LytX truncates, CbpX truncate-LytX truncate chimeric proteins, pneumolysin (Ply), PspA, PsaA, Sp128, Sp101, Sp130, Sp125 or Sp133.

*Streptococcus pyogenes (Group A Streptococcus):* Group A Streptococcus antigens may include a protein identified in WO 02/34771 or WO 2005/032582 (including GAS 40), fusions of fragments of GAS M proteins (including those described in WO 02/094851, and Dale, Vaccine (1999) 17:193-200, and Dale, Vaccine 14(10): 944-948), fibronectin binding protein (Sfb1), Streptococcal heme-associated protein (Shp), and Streptolysin S (SagA).

*Moraxella catarrhalis:* Moraxella antigens include antigens identified in WO 02/18595 and WO 99/58562, outer membrane protein antigens (HMW-OMP), C-antigen, and/or LPS.

*Bordetella pertussis:* Pertussis antigens include petussis holotoxin (PT) and filamentous haemagglutinin (FHA) from *B. pertussis,* optionally also combination with pertactin and/or agglutinogens 2 and 3 antigen.

Pertussis antigen ('P') can be either cellular ('wP') or acellular ('aP'). Cellular pertussis antigens typically take the form of inactivated *B.pertussis* cells. Preparation of cellular pertussis antigens is well documented *[e.g.* see chapter 21 of Vaccines, eds. Plotkin & Orenstein, 4th edition, 2004] *e.g.* it may be obtained by heat inactivation of phase I culture *of B.pertussis.* Quantities of wP antigens can be expressed in international units (IU). For example, the NIBSC supplies the 'Third International Standard For Pertussis Vaccine' [NIBSC code: 66/303], which contains 46 IU per ampoule. Each ampoule contains the freeze-dried residue of 2.0 ml aliquots of an aqueous solution which contained 10 liters of bacterial suspension (equivalent to ISO opacity units in terms of the U.S. Opacity Standard) diluted with eight litres of M/15 Sorensen's buffer pH 7.0. As an alternative to the IU system, the 'OU' unit ("opacity units") is also used *(e.g.* 4 OU may be about 1 IU). *Acellular* pertussis antigens currently used in vaccines include pertussis toxoid (PT), filamentous haemagglutinin (FHA). pertactin (also known as the '69 kiloDalton outer membrane protein'), and fimbriae *(e.g.* agglutinogens 2 and 3), The invention preferably uses at least two of, and preferably all three of, PT, FHA and pertactin *(i.e.* without using fimbriae). These three antigens are preferably prepared by isolation from *.ft.pertussis* culture grown in modified Slainer-Scholte liquid medium. PT and FHA can be isolated from the fermentation broth *(e.g.* by adsorption on hydroxyapatite gel), whereas pertactin can be extracted from the cells by heat treatment and flocculation *(e.g.* using barium chloride). The antigens can be purified in successive chromatographic and/or precipitation steps. PT and FHA can be purified by hydrophobia chromatography, affinity chromatography and size exclusion chromatography. Pertactin can be purified by ion exchange cliromatography, hydrophobia chromatography and size exclusion chromatography. FI-IA and pertactin may be treated with formaldehyde prior to use according to the invention. FP is preferably detoxified by treatment with formaldehyde and/or glutaraldehydc. As an alternative to this chemical detoxification procedure the PT may be a mutant PT in which enzymatic activity has been reduced by mutagenesis [Roppuoli et al., 1991, TIBTECH 9: 232-238], but detoxification by chemical treatment is preferred. Quantities of acelfular pertussis antigens are typically expressed in micrograms.

*Staphylococcus* aureus: Staph aureus antigens include S. *aureus* type 5 and 8 capsular polysaccharides optionally conjugated to nontoxic recombinant *Pseudomonas aeruginosa* exotoxin A, such as StaphVAX™, or antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin).

*Staphylococcus epidermis: S. epidermidis* antigens include slime-associated antigen (SAA).

*Clostridium tetani* (Tetanus): Tetanus antigens include tetanus toxoid (TT), preferably used as a carrier protein in conjunction/conjugated with the compositions of the present invention.

*Cornynebacterium diphtheriae* (Diphtheria): Diphtheria antigens include diphtheria toxin, preferably detoxified, such as CRM₁₉₇. Additionally antigens capable of modulating, inhibiting or associated with ADP ribosylation are contemplated for combination/co-administration/conjugation with the compositions of the present invention. The diphtheria toxoids may be used as carrier proteins.

*Haemophilus influenzae B (Hib):* Hib antigens include a Hib saccharide antigen.

*Pseudomonas aeruginosa:* Pseudomonas antigens include endotoxin A, Wzz protein, *P. aeruginosa* LPS, more particularly LPS isolated from PAO1 (05 serotype), and/or Outer Membrane Proteins, including Outer Membrane Proteins F (OprF) *(*Infect Immun. 2001 May; 69(5): 3510-3515).

*Legionella pneumophila.* Bacterial antigens may be derived from Legionella pneumophila.

*Streptococcus agalactiae (Group B Streptococcus)*: Group B Streptococcus antigens include a protein or saccharide antigen identified in WO 02/34771, WO 03/093306, WO 04/041157, or WO 2005/002619 (including proteins GBS 80, GBS 104, GBS 276 and GBS 322, and including saccharide antigens derived from serotypes Ia, Ib, Ia/c, II, III, IV, V, VI, VII and VIII).

*Neiserria gonorrhoeae:* Gonorrhoeae antigens include Por (or porin) protein, such as PorB (*see* Zhu et al., Vaccine (2004) 22:660 - 669), a transferring binding protein, such as TbpA and TbpB (See Price et al., Infection and Immunity (2004) 71(1):277 - 283), a opacity protein (such as Opa), a reduction-modifiable protein (Rmp), and outer membrane vesicle (OMV) preparations (see Plante et al., J Infectious Disease (2000) 182:848 - 855), also see e.g. WO99/24578, WO99/36544, WO99/57280, WO02/079243).

*Chlamydia trachomatis:* Chlamydia trachomatis antigens include antigens derived from serotypes A, B, Ba and C (agents of trachoma, a cause of blindness), serotypes L₁, L₂ & L₃ (associated with Lymphogranuloma venereum), and serotypes, D-K. Chlamydia trachomas antigens may also include an antigen identified in WO 00/37494, WO 03/049762, WO 03/068811, or WO 05/002619, including PepA (CT045), LcrE (CT089), ArtJ (CT381), DnaK (CT396), CT398, OmpH-like (CT242), L7/L12 (CT316), OmcA (CT444), AtosS (C467), CT547, Eno (CT587), HrtA (CT823), and MurG (CT761).

*Treponema pallidum* (Syphilis): Syphilis antigens include TmpA antigen.

*Haemophilus ducreyi* (causing chancroid): Ducreyi antigens include outer membrane protein (DsrA).

*Enterococcus faecalis* or *Enterococcus faecium:* Antigens include a trisaccharide repeat or other *Enterococcus* derived antigens provided in US Patent No. 6,756,361.
*Helicobacter pylori:* H pylori antigens include Cag, Vac, Nap, HopX, HopY and/or urease antigen.

*Staphylococcus saprophyticus:* Antigens include the 160 kDa hemagglutinin of *S. saprophyticus* antigen.

*Yersinia enterocolitica* Antigens include LPS (Infect Immun. 2002 August; 70(8): 4414).

*E. coli:* E. coli antigens may be derived from enterotoxigenic *E. coli* (ETEC), enteroaggregative E. *coli* (EAggEC), diffusely adhering *E. coli* (DAEC), enteropathogenic *E. coli* (EPEC), and/or enterohemorrhagic *E. coli* (EHEC).

*Bacillus anthracis* (anthrax): *B. anthracis* antigens are optionally detoxified and may be selected from A-components (lethal factor (LF) and edema factor (EF)), both of which can share a common B-component known as protective antigen (PA).

*Yersinia pestis* (plague): Plague antigens include F1 capsular antigen (Infect Immun. 2003 Jan; 71(1)): 374-383, LPS (Infect Immun. 1999 Oct; 67(10): 5395), Yersinia pestis V antigen (Infect Immun. 1997 Nov; 65(11): 4476-4482).

Mycobacterium tuberculosis: *Tuberculosis antigens include lipoproteins, LPS, BCG antigens,* a *fusion proteins of antigen 85B (Ag85B) and*/*or ESAT-6 optionally formulated in cationic lipid vesicles* (Infect Immun. 2004 October; 72(10): 6148*), Mycobacterium tuberculosis (Mtb) isocitrate dehydrogenase associated antigens* (Proc Natl Acad Sci USA. 2004 Aug 24; 101(34): 12652), and/or MPT51 antigens (Infect Immun. 2004 July; 72(7): 3829*).*

Rickettsia: Antigens include outer membrane proteins, including the outer membrane protein A and/or B (OmpB) *(*Biochim Biophys Acta. 2004 Nov 1;1702(2):145), LPS, and surface protein antigen (SPA) (J Autoimmun. 1989 Jun;2 Suppl:81).

*Listeria monocytogenes.* Bacterial antigens may be derived from Listeria monocytogenes.

*Chlamydia pneumoniae:* Antigens include those identified in WO 02/02606.

*Vibrio cholerae:* Antigens include proteinase antigens, LPS, particularly lipopolysaccharides of Vibrio cholerae II, O1 Inaba O-specific polysaccharides, V. cholera 0139, antigens of IEM108 vaccine *(*Infect Immun. 2003 Oct:71(10):5498-504), and/or Zonula occludens toxin (Zot).

*Salmonella typhi* (typhoid fever): Antigens include capsular polysaccharides preferably conjugates (Vi, i.e. vax-TyVi).

*Borrelia burgdorferi* (Lyme disease): Antigens include lipoproteins (such as OspA, OspB, Osp C and Osp D), other surface proteins such as OspE-related proteins (Erps), decorin-binding proteins (such as DbpA), and antigenically variable VI proteins. , such as antigens associated with P39 and P13 (an integral membrane protein, Infect Immun. 2001 May; 69(5): 3323-3334), VlsE Antigenic Variation Protein (J Clin Microbiol. 1999 Dec; 37(12): 3997).

*Porphyromonas gingivalis:* Antigens include *P. gingivalis* outer membrane protein (OMP).

*Klebsiella:* Antigens include an OMP, including OMP A, or a polysaccharide optionally conjugated to tetanus toxoid.

Further bacterial antigens of the invention may be capsular antigens, polysaccharide antigens or protein antigens of any of the above. Further bacterial antigens may also include an outer membrane vesicle (OMV) preparation. Additionally, antigens include live, attenuated, and/or purified versions of any of the aforementioned bacteria. The antigens of the present invention may be derived from gram-negative or gram-positive bacteria. The antigens of the present invention may be derived from aerobic or anaerobic bacteria.

Additionally, any of the above bacterial-derived saccharides (polysaccharides, LPS, LOS or oligosaccharides) can be conjugated to another agent or antigen, such as a carrier protein (for example CRM₁₉₇). Such conjugation may be direct conjugation effected by reductive amination of carbonyl moieties on the saccharide to amino groups on the protein, as provided in US Patent No. 5,360,897 and Can J Biochem Cell Biol. 1984 May:62(5):270-5. Alternatively, the saccharides can be conjugated through a linker, such as, with succinamide or other linkages provided in Bioconjugate Techniques, 1996 and CRC, Chemistry of Protein Conjugation and Cross-Linking, 1993.

### B. Viral Antigens

Viral antigens suitable for use in the invention include inactivated (or killed) virus, attenuated virus, split virus formulations, purified subunit formulations, viral proteins which may be isolated, purified or derived from a virus, and Virus Like Particles (LPS). Viral antigens may be derived from viruses propagated on cell culture or other substrate. Alternatively, viral antigens may be expressed recombinantly. Viral antigens preferably include epitopes which are exposed on the surface of the virus during at least one stage of its life cycle. Viral antigens are preferably conserved across multiple serotypes or isolates. Viral antigens include antigens derived from one or more of the viruses set forth below as well as the specific antigens examples identified below.

*Orthomyxovirus:* Viral antigens may be derived from an Orthomyxovirus, such as Influenza A, B and C. Orthomyxovirus antigens may be selected from one or more of the viral proteins, including hemagglutinin (HA), neuraminidase (NA), nucleoprotein (NP), matrix protein (M1), membrane protein (M2), one or more of the transcriptase components (PB1, PB2 and PA). Preferred antigens include HA and NA.

Influenza antigens may be derived from interpandemic (annual) flu strains. Alternatively influenza antigens may be derived from strains with the potential to cause pandemic a pandemic outbreak (i.e., influenza strains with new haemagglutinin compared to the haemagglutinin in currently circulating strains, or influenza strains which are pathogenic in avian subjects and have the potential to be transmitted horizontally in the human population, or influenza strains which are pathogenic to humans).

*Paramyxoviridae* viruses: Viral antigens may be derived from Paramyxoviridae viruses, such as Pneumoviruses (RSV), Paramyxoviruses (PIV) and Morbilliviruses (Measles).

*Pneumovirus:* Viral antigens may be derived from a Pneumovirus, such as Respiratory syncytial virus (RSV), Bovine respiratory syncytial virus, Pneumonia virus of mice, and Turkey rhinotracheitis virus. Preferably, the Pneumovirus is RSV. Pneumovirus antigens may be selected from one or more of the following proteins, including surface proteins Fusion (F), Glycoprotein (G) and Small Hydrophobic protein (SH), matrix proteins M and M2, nucleocapsid proteins N, P and L and nonstructural proteins NS1 and NS2. Preferred Pneumovirus antigens include F, G and M. See e.g., J Gen Virol. 2004 Nov; 85(Pt 11):3229). Pneumovirus antigens may also be formulated in or derived from chimeric viruses. For example, chimeric RSV/PIV viruses may comprise components of both RSV and PIV. *Paramyxovirus:* Viral antigens may be derived from a Paramyxovirus; such as Parainfluenza virus types 1 - 4 (PIV), Mumps, Sendai viruses, Simian virus 5, Bovine parainfluenza virus and Newcastle disease virus. Preferably, the Paramyxovirus is PIV or Mumps. Paramyxovirus antigens may be selected from one or more of the following proteins: Hemagglutinin -Neuraminidase (HN), Fusion proteins F1 and F2, Nucleoprotein (NP), Phosphoprotein (P), Large protein (L), and Matrix protein (M). Preferred Paramyxovirus proteins include HN, F1 and F2. Paramyxovirus antigens may also be formulated in or derived from chimeric viruses. For example, chimeric RSV/PIV viruses may comprise components of both RSV and PIV. Commercially available mumps vaccines include live attenuated mumps virus, in either a monovalent form or in combination with measles and rubella vaccines (MMR).

*Morbillivirus:* Viral antigens may be derived from a Morbillivirus, such as Measles. Morbillivirus antigens may be selected from one or more of the following proteins: hemagglutinin (H), Glycoprotein (G), Fusion factor (F), Large protein (L), Nucleoprotein (NP), Polymerase phosphoprotein (P), and Matrix (M). Commercially available measles vaccines include live attenuated measles virus, typically in combination with mumps and rubella (MMR).

*Picornavirus:* Viral antigens may be derived from Picornaviruses, such as Enteroviruses, Rhinoviruses, Heparnavirus, Cardioviruses and Aphthoviruses. Antigens derived from Enteroviruses, such as Poliovirus are preferred.

*Enterovirus:* Viral antigens may be derived from an Enterovirus, such as Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71. Preferably, the Enterovirus is poliovirus. Enterovirus antigens are preferably selected from one or more of the following Capsid proteins VP1, VP2, VP3 and VP4. Commercially available polio vaccines include InactivatEd Polio Vaccine (IPV) and Oral poliovirus vaccine (OPV).

*Heparnavirus*: Viral antigens may be derived from an Heparnavirus, such as Hepatitis A virus (HAV). Commercially available HAV vaccines include inactivated HAV vaccine.

*Togavirus*: Viral antigens may be derived from a Togavirus, such as a Rubivirus, an Alphavirus, or an Arterivirus. Antigens derived from Rubivirus, such as Rubella virus, are preferred. Togavirus antigens may be selected from E1, E2, E3, C, NSP-1, NSPO-2, NSP-3 or NSP-4. Togavirus antigens are preferably selected from E1, E2 or E3. Commercially available Rubella vaccines include a live cold-adapted virus, typically in combination with mumps and measles vaccines (MMR).

*Flavivirus*: Viral antigens may be derived from a Flavivirus, such as Tick-borne encephalitis (TBE), Dengue (types 1, 2, 3 or 4), Yellow Fever, Japanese encephalitis, West Nile encephalitis, St. Louis encephalitis, Russian spring-summer encephalitis, Powassan encephalitis. Flavivirus antigens may be selected from PrM, M, C, E, NS-1, NS-2a, NS2b, NS3, NS4a, NS4b, and NS5. Flavivirus antigens are preferably selected from PrM, M and E. Commercially available TBE vaccine include inactivated virus vaccines.

*Pestivirus*: Viral antigens may be derived from a Pestivirus, such as Bovine viral diarrhea (BVDV), Classical swine fever (CSFV) or Border disease (BDV).

*Hepadnavirus*: Viral antigens may be derived from a Hepadnavirus, such as Hepatitis B virus. Hepadnavirus antigens may be selected from surface antigens (L, M and S), core antigens (HBc, HBe). Commercially available HBV vaccines include subunit vaccines comprising the surface antigen S protein.

*Hepatitis C virus*: Viral antigens may be derived from a Hepatitis C virus (HCV). HCV antigens may be selected from one or more of E1, E2, E1/E2, NS345 polyprotein, NS 345-core polyprotein, core, and/or peptides from the nonstructural regions (Houghton et al., Hepatology (1991) 19:381).

*Rhabdovirus*: Viral antigens may be derived from a Rhabdovirus, such as a Lyssavirus (Rabies virus) and Vesiculovirus (VSV). Rhabdovirus antigens may be selected from glycoprotein (G), nucleoprotein (N), large protein (L), nonstructural proteins (NS). Commercially available Rabies virus vaccine comprise killed virus grown on human diploid cells or fetal rhesus lung cells.

*Caliciviridae;* Viral antigens may be derived from Calciviridae, such as Norwalk virus, and Norwalk-like Viruses, such as Hawaii Virus and Snow Mountain Virus.

*Coronavirus:* Viral antigens may be derived from a Coronavirus, SARS, Human respiratory coronavirus, Avian infectious bronchitis (IBV), Mouse hepatitis virus (MHV), and Porcine transmissible gastroenteritis virus (TGEV). Coronavirus antigens may be selected from spike (S), envelope (E), matrix (M), nucleocapsid (N), and Hemagglutinin-esterase glycoprotein (HE). Preferably, the Coronavirus antigen is derived from a SARS virus. SARS viral antigens are described in WO 04/92360;

Retrovirus: Viral antigens may be derived from a Retrovirus, such as an Oncovirus, a Lentivirus or a Spumavirus. Oncovirus antigens may be derived from HTLV-1, HTLV-2 or HTLV-5. Lentivirus antigens may be derived from HIV-1 or HIV-2. Retrovirus antigens may be selected from gag, pol, env, tax, tat, rex, rev, nef, vif, vpu, and vpr. HIV antigens may be selected from gag (p24gag and p55gag), env (gp160 and gp41), pol, tat, nef, rev vpu, miniproteins, (preferably p55 gag and gp140v delete). HIV antigens may be derived from one or more of the following strains: HIV_{IIIb}, HIV_{SF2}, HIV_{LAV}, HIV_{LAI}, HIV_{MN}, HIV-1_{CM235}, HIV-1_{US4}.

*Reovirus*: Viral antigens may be derived from a Reovirus, such as an Orthoreovirus, a Rotavirus, an Orbivirus, or a Coltivirus. Reovirus antigens may be selected from.structural proteins λ1, λ2, λ3, µ1, µ2, σ1, σ2, or σ3, or nonstructural proteins σNS, µNS, or σ1s. Preferred Reovirus antigens may be derived from a Rotavirus. Rotavirus antigens may be selected from VP1, VP2, VP3, VP4 (or the cleaved product VP5 and VP8), NSP 1, VP6, NSP3, NSP2, VP7, NSP4, or NSP5. Preferred Rotavirus antigens include VP4 (or the cleaved product VP5 and VP8), and VP7.

*Parvovirus:* Viral antigens may be derived from a Parvovirus, such as Parvovirus B19. Parvovirus antigens may be selected from VP-1, VP-2, VP-3, NS-1 and NS-2. Preferably, the Parvovirus antigen is capsid protein VP-2.

*Delta hepatitis virus (HDV):* Viral antigens may be derived HDV, particularly δ-antigen from HDV (see, e.g., U.S. Patent No. 5,378,814).

*Hepatitis E virus (HEV)*: Viral antigens may be derived from HEV.

*Hepatitis G virus (HGV)*: Viral antigens may be derived from HGV.

*Human Herpesvirus*: Viral antigens may be derived from a Human Herpesvirus, such as Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8). Human Herpesvirus antigens may be selected from immediate early proteins (α), early proteins (β), and late proteins (γ). HSV antigens may be derived from HSV-1 or HSV-2 strains. HSV antigens may be selected from glycoproteins gB, gC, gD and gH, fusion protein (gB), or immune escape proteins (gC, gE, or gI). VZV antigens may be selected from core, nucleocapsid, tegument, or envelope proteins. A live attenuated VZV vaccine is commercially available. EBV antigens may be selected from early antigen (EA) proteins, viral capsid antigen (VCA), and glycoproteins of the membrane antigen (MA). CMV antigens may be selected from capsid proteins, envelope glycoproteins (such as gB and gH), and tegument proteins

*Papovaviruses*: Antigens may be derived from Papovaviruses, such as Papillomaviruses and Polyomaviruses. Papillomaviruses include HPV serotypes 1, 2, 4, 5, 6, 8, 11, 13, 16, 18, 31, 33, 35, 39, 41, 42, 47, 51, 57, 58, 63 and 65. Preferably, HPV antigens are derived from serotypes 6, 11, 16 or 18. HPV antigens may be selected from capsid proteins (L1) and (L2), or E1 - E7, or fusions thereof. HPV antigens are preferably formulated into virus-like particles (VLPs). Polyomyavirus viruses include BK virus and JK virus. Polyomavirus antigens may be selected from VP1, VP2 or VP3.

Further provided are antigens, compositions, methods, and microbes included in Vaccines, 4th Edition (Plotkin and Orenstein ed. 2004); Medical Microbiology 4th Edition (Murray et al. ed. 2002); Virology, 3rd Edition (W.K. Joklik ed. 1988); Fundamental Virology, 2nd Edition (B.N. Fields and D.M. Knipe, eds. 1991), which are contemplated in conjunction with the compositions of the present invention.

### C. Fungal Antigens

Fungal antigens for use in the invention may be derived from one or more of the fungi set forth below.

Fungal antigens may be derived from Dermatophytres, including: *Epidermophyton floccusum, Microsporum audouini, Microsporum canis, Microsporum distortum, Microsporum equinum, Microsporum gypsum, Microsporum nan um, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme.*

Fungal pathogens may be derived from *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei,* Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp, and Cladosporium spp.

Processes for producing a fungal antigens are well known in the art (see US Patent No. 6,333,164). In a preferred method a solubilized fraction extracted and separated from an insoluble fraction obtainable from fungal cells of which cell wall has been substantially removed or at least partially removed, characterized in that the process comprises the steps of: obtaining living fungal cells; obtaining fungal cells of which cell wall has been substantially removed or at least partially removed; bursting the fungal cells of which cell wall has been substantially removed or at least partially removed; obtaining an insoluble fraction; and extracting and separating a solubilized fraction from the insoluble fraction.

### D. STD Antigens

The compositions of the invention may include one or more antigens derived from a sexually transmitted disease (STD). Such antigens may provide for prophylactis or therapy for STD's such as chlamydia, genital herpes, hepatits (such as HCV), genital warts,' gonorrhoea, syphilis and/or chancroid (See, WO00/15255). Antigens may be derived from one or more viral or bacterial STD's. Viral STD antigens for use in the invention may be derived from, for example, HIV, herpes simplex virus (HSV-1 and HSV-2), human papillomavirus (HPV), and hepatitis (HCV). Bacterial STD antigens for use in the invention may be derived from, for example, *Neiserria gonorrhoeae, Chlamydia trachomatis, Treponema pallidum, Haemophilus ducreyi*, *E. coli,* and *Streptococcus agalactiae.* Examples of specific antigens derived from these pathogens are described above.

### E. Respiratory Antigens

The compositions of the invention may include one or more antigens derived from a pathogen which causes respiratory disease. For example, respiratory antigens may be derived from a respiratory virus such as Orthomyxoviruses (influenza), Pneumovirus (RSV), Paramyxovirus (PIV), Morbillivirus (measles), Togavirus (Rubella), VZV, and Coronavirus (SARS). Respiratory antigens may be derived from a bacteria which causes respiratory disease, such as *Streptococcus pneumoniae, Pseudomonas aeruginosa, Bordetella pertussis, Mycobacterium tuberculosis, Mycoplasma pneumoniae, Chlamydia pneumoniae, Bacillus anthracis,* and *Moraxella catarrhalis.* Examples of specific antigens derived from these pathogens are described above.

### F. Pediatric Vaccine Antigens

The compositions of the invention may include one or more antigens suitable for use in pediatric subjects. Pediatric subjects are typically less than about 3 years old, or less than about 2 years old, or less than about 1 years old. Pediatric antigens may be administered multiple times over the course of 6 months, 1, 2 or 3 years. Pediatric antigens may be derived from a virus which may target pediatric populations and/or a virus from which pediatric populations are susceptible to infection. Pediatric viral antigens include antigens derived from one or more of Orthomyxovirus (influenza), Pneumovirus. (RSV), Paramyxovirus (PIV and Mumps), Morbillivirus (measles), Togavirus (Rubella), Enterovirus (polio), HBV, Coronavirus (SARS), and Varicella-zoster virus (VZV), Epstein Barr virus (EBV). Pediatric bacterial antigens include antigens derived from one or more of *Streptococcus pneumoniae, Neisseria meningitides, Streptococcus pyogenes* (Group A Streptococcus), *Moraxella catarrhalis, Bordetella pertussis, Staphylococcus aureus, Clostridium tetani* (Tetanus), *Cornynebacterium diphtheriae* (Diphtheria), *Haemophilus influenzae B* (Hib), *Pseudomonas aeruginosa, Streptococcus agalactiae* (Group B Streptococcus), and *E*. *coli.* Examples of specific antigens derived from these pathogens are described above.

### G. Antigens suitable for use in Elderly or Immunocompromised Individuals

The compositions of the invention may include one or more antigens suitable for use in elderly or immunocompromised individuals. Such individuals may need to be vaccinated more frequently, with higher doses or with adjuvanted formulations to improve their immune response to the targeted antigens. Antigens which may be targeted for use in Elderly or Immunocompromised individuals include antigens derived from one or more of the following pathogens: Neisseria meningitides, Streptococcus pneumoniae, Streptococcus pyogenes (*Group A Streptococcus)*, Moraxella catarrhalis, Bordetella pertussis, Staphylococcus aureus, Staphylococcus epidermis, Clostridium tetani *(Tetanus)*, Cornynebacterium diphtheriae *(Diphtheria)*, Haemophilus influenzae B *(Hib)*, Pseudomonas aeruginosa, Legionella pneumophila, Streptococcus agalactiae *(Group B Streptococcus)*, Enterococcus faecalis, Helicobacter pylori, Clamydia pneumoniae, *Orthomyxovirus (influenza)*, *Pneumovirus (RSV), Paramyxovirus (PIV and Mumps), Morbillivirus (measles), Togavirus (Rubella), Enterovirus (polio), HBV, Coronavirus (SARS), Varicella-zoster virus (VZV), Epstein Barr virus (EBV), Cytomegalovirus (CMV). Examples of specific antigens derived from these pathogens are described above*.

### H. Antigens suitable for use in Adolescent Vaccines

The compositions of the invention may include one or more antigens suitable for use in adolescent subjects. Adolescents may be in need of a boost of a previously administered pediatric antigen. Pediatric antigens which may be suitable for use in adolescents are described above. In addition, adolescents may be targeted to receive antigens derived from an STD pathogen in order to ensure protective or therapeutic immunity before the beginning of sexual activity. STD antigens which may be suitable for use in adolescents are described above.

### I. Antigen Formulations

In other aspects of the invention, methods of producing microparticles having adsorbed antigens are provided. The methods comprise: (a) providing an emulsion by dispersing a mixture comprising (i) water, (ii) a detergent, (iii) an organic solvent, and (iv) a biodegradable polymer selected from the group consisting of a poly(α-hydroxy acid), a polyhydroxy butyric acid, a polycaprolactone, a polyorthoester, a polyanhydride, and a polycyanoacrylate. The polymer is typically present in the mixture at a concentration of about 1% to about 30% relative to the organic solvent, while the detergent is typically present in the mixture at a weight-to-weight detergent-to-polymer ratio of from about 0.00001:1 to about 0.1:1 (more typically about 0.0001:1 to about 0.1:1, about 0.001:1 to about 0.1:1, or about 0.005:1 to about 0.1:1); (b) removing the organic solvent from the emulsion; and (c) adsorbing an antigen on the surface of the microparticles. In certain embodiments, the biodegradable polymer is present at a concentration of about 3% to about 10% relative to the organic solvent.

Microparticles for use herein will be formed from materials that are sterilizable, non-toxic and biodegradable. Such materials include, without limitation, poly(α-hydroxy acid), polyhydroxybutyric acid, polycaprolactone, polyorthoester, polyanhydride, PACA, and polycyanoacrylate. Preferably, microparticles for use with the present invention are derived from a poly(α-hydroxy acid), in particular, from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials which have a variety of molecular weights and, in the case of the copolymers such as PLG, a variety of lactide:glycolide ratios, the selection of which will be largely a matter of choice, depending in part on the coadministered macromolecule. These parameters are discussed more fully below.

Further antigens may also include an outer membrane vesicle (OMV) preparation.

Additional formulation methods and antigens (especially tumor antigens) are provided in U.S. Patent Serial No. 09/581,772.

### J. Antigen References

The following references include antigens useful in conjunction with the compositions of the present invention:
1 International patent application WO99/24578
2 International patent application WO99/36544.
3 International patent application WO99/57280.
4 International patent application WO00/22430.
5 Tettelin et al. (2000) Science 287:1809-1815.
6 International patent application WO96/29412.
7 Pizza et al. (2000) Science 287:1816-1820.
8 PCT WO 01/52885.
9 Bjune et al. (1991) Lancet 338(8775).
10 Fuskasawa et al. (1999) Vaccine 17:2951-2958.
11 Rosenqist et al. (1998) Dev. Biol. Strand 92:323-333.
12 Constantino et al. (1992) Vaccine 10:691-698.
13 Constantino et al. (1999) Vaccine 17:1251-1263.
14 Watson (2000) Pediatr Infect Dis J 19:331-332.
15 Rubin (20000) Pediatr Clin North Am 47:269-285,v.
16 Jedrzejas (2001) Microbiol Mol Biol Rev 65:187-207.
17 International patent application filed on 3^{rd} July 2001 claiming priority from GB-0016363.4;WO 02/02606; PCT IB/01/00166.
18 Kalman et al. (1999) Nature Genetics 21:385-389.
19 Read et al. (2000) Nucleic Acids Res 28:1397-406.
20Shirai et al. (2000) J. Infect. Dis 181 (Suppl 3):S524-S527.
21 International patent application WO99/27105.
22 International patent application WO00/27994.
23 International patent application WO00/37494.
24 International patent application WO99/28475.
25 Bell (2000) Pediatr Infect Dis J 19:1187-1188.
26 Iwarson (1995) APMIS 103:321-326.
27Gerlich et al. (1990) Vaccine 8 Suppl:S63-68 & 79-80.
28 Hsu et al. (1999) Clin Liver Dis 3:901-915.
29 Gastofsson et al. (1996) N. Engl. J. Med. 334-:349-355.
30Rappuoli et al. (1991) TIBTECH 9:232-238.
31 Vaccines (1988) eds. Plotkin & Mortimer. ISBN 0-7216-1946-0.
32 Del Guidice et al. (1998) Molecular Aspects of Medicine 19:1-70.
33 International patent application WO93/018150.
34 International patent application WO99/53310.
35 International patent application WO98/04702.
36 Ross et al. (2001) Vaccine 19:135-142.
37 Sutter et al. (2000) Pediatr Clin North Am 47:287-308.
38 Zimmerman & Spann (1999) Am Fan Physician 59:113-118, 125-126.
39Dreensen (1997) Vaccine 15 Suppl "52-6.
40MMWR Morb Mortal Wkly rep 1998 Jan 16:47(1):12, 9.
41 McMichael (2000) Vaccine19 Suppl 1:S101-107.
42 Schuchat (1999) Lancer 353(9146):51-6.
43 GB patent applications 0026333.5, 0028727.6 & 0105640.7.
44 Dale (1999) Infect Disclin North Am 13:227-43, viii.
45 Ferretti et al. (2001) PNAS USA 98: 4658-4663.
46 Kuroda et al. (2001) Lancet 357(9264):1225-1240; see also pages 1218-1219.
47 Ramsay et al. (2001) Lancet 357(9251):195-196.
48Lindberg (1999) Vaccine 17 Suppl 2:S28-36.
49 Buttery & Moxon (2000) J R Coil Physicians Long 34:163-168.
50Ahmad & Chapnick (1999) Infect Dis Clin North Am 13:113-133, vii.
51 Goldblatt (1998) J. Med. Microbiol. 47:663-567.
52 European patent 0 477 508.
53 U.S. Patent No. 5,306,492.
54 International patent application WO98/42721.
55 Conjugate Vaccines (eds. Cruse et al.) ISBN 3805549326, particularly vol. 10:48-114.
56 Hermanson (1996) Bioconjugate Techniques ISBN: 012323368 & 012342335X.
57 European patent application 0372501.
58 European patent application 0378881.
59 European patent application 0427347.
60 International patent application WO93/17712.
61 International patent application WO98/58668.
62 European patent application 0471177.
63 International patent application WO00/56360.
64 International patent application WO00/67161.

Compositions of the invention will typically, in addition to the components mentioned above, comprise one or more "pharmaceutically acceptable carriers." These include any carrier which does not itself induce the production of antibodies harmful to the individual receiving the composition. Suitable carriers typically are large, slowly metabolized macromolecules such as proteins, polysaccharides, polylactic acids, polyglycolic acids, polymeric amino acids, amino acid copolymers, and lipid aggregates (such as oil droplets or liposomes). Such carriers are well known to those of ordinary skill in the art. A composition may also contain a diluent, such as water, saline, glycerol, etc. Additionally, an auxiliary substance, such as a wetting or emulsifying agent, pH buffering substance, and the like, may be present. A thorough discussion of pharmaceutically acceptable components is available in Gennaro (2000) Remington: The Science and Practice of Pharmacy. 20th ed., ISBN: 0683306472.

The immunogenic compositions of the invention may be prepared in various forms. For example, the compositions may be prepared as injectables, either as liquid solutions or suspensions. Solid forms suitable for solution in, or suspension in, liquid vehicles prior to injection can also be prepared (*e.g*. a lyophilised composition or a spray-freeze dried composition). The composition may be prepared for topical administration e.g. as an ointment, cream or powder. The composition may be prepared for oral administration *e.g.* as a tablet or capsule, as a spray, or as a syrup (optionally flavoured). The composition may be prepared for pulmonary administration e.g. as an inhaler, using a fine powder or a spray. The composition may be prepared as a suppository or pessary. The composition may be prepared for nasal, aural or ocular administration e.g. as drops. The composition may be in kit form, designed such that a combined composition is reconstituted just prior to administration to a patient. Such kits may comprise one or more antigens in liquid form and one or more lyophilised antigens.

Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

Immunogenic compositions of the present invention may be administered in conjunction with other immunoregulatory agents. For example, a vaccine of the invention can include an adjuvant. Preferred adjuvants include, but are not limited to, one or more of the following types of adjuvants described below.

### A. Mineral Containing Compositions

Mineral containing compositions suitable for use as adjuvants in the invention include mineral salts, such as aluminum salts and calcium salts. The invention includes mineral salts such as hydroxides (*e.g*. oxyhydroxides), phosphates (*e.g*. hydroxyphosphates, orthophosphates), sulfates, *etc*. (*e.g*. see chapters 8 & 9 of Vaccine Design... (1995) eds. Powell & Newman. ISBN: 030644867X. Plenum.), or mixtures of different mineral compounds (*e.g*. a mixture of a phosphate and a hydroxide adjuvant, optionally with an excess of the phosphate), with the compounds taking any suitable form (*e.g*. gel, crystalline, amorphous, *etc*.), and with adsorption to the salt(s) being preferred. The mineral containing compositions may also be formulated as a particle of metal salt (WO00/23105).

Aluminum salts may be included in vaccines of the invention such that the dose of Al³⁺ is between 0.2 and 1.0 mg per dose. In one embodiment the aluminum based adjuvant for use in the present invention is alum (aluminum potassium sulfate (AlK(SO₄)₂)), or an alum derivative, such as that formed insitu by mixing an antigen in phosphate buffer with alum, followed by titration and precipitation with a base such as ammonium hydroxide or sodium hydroxide.

Another aluminum-based adjuvant for use in vaccine formulations of the present invention is aluminum hydroxide adjuvant (Al(OH)₃) or crystalline aluminum oxyhydroxide (AlOOH), which is an excellent adsorbant, having a surface area of approximately 500m²/g. Alternatively, aluminum phosphate adjuvant (Al-PO₄) or aluminum hydroxyphosphate, which contains phosphate groups in place of some or all of the hydroxyl groups of aluminum hydroxide adjuvant is provided. Preferred aluminum phosphate adjuvants provided herein are amorphous and soluble in acidic, basic and neutral media.

In another embodiment the adjuvant of the invention comprises both aluminum phosphate and aluminum hydroxide. In a more particular embodiment thereof, the adjuvant has a greater amount of aluminum phosphate than aluminum hydroxide, such as a ratio of 2:1, 3:1, 4:1, 5:1, 6:1, 7:1, 8:1, 9:1 or greater than 9:1, by weight aluminum phosphate to aluminum hydroxide. More particular still, aluminum salts in the vaccine are present at 0.4 to 1.0 mg per vaccine dose, or 0.4 to 0.8 mg per vaccine dose, or 0.5 to 0.7 mg per vaccine dose, or about 0.6 mg per vaccine dose.

Generally, the preferred aluminum-based adjuvant(s), or ratio of multiple aluminum-based adjuvants, such as aluminum phosphate to aluminum hydroxide is selected by optimization of electrostatic attraction between molecules such that the antigen carries an opposite charge as the adjuvant at the desired pH. For example, aluminum phosphate adjuvant (iep = 4) adsorbs lysozyme, but not albumin at pH 7.4. Should albumin be the target, aluminum hydroxide adjuvant would be selected (iep 11.4). Alternatively, pretreatment of aluminum hydroxide with phosphate lowers its isoelectric point, making it a preferred adjuvant for more basic antigens.

### B. Oil-Emulsions

Oil-emulsion compositions suitable for use as adjuvants in the invention include squalene-water emulsions, such as MF59 (5% Squalene, 0.5% Tween 80, and 0.5% Span 85, formulated into submicron particles using a microfluidizer). See WO90/14837. See also, Podda, "The adjuvanted influenza vaccines with novel adjuvants: experience with the MF59-adjuvanted vaccine", Vaccine (2001) 19: 2673-2680; Frey et al., "Comparison of the safety, tolerability, and immunogenicity of a MF59-adjuvanted influenza vaccine and a non-adjuvanted influenza vaccine in non-elderly adults", Vaccine (2003) 21:4234-4237. MF59 is used as the adjuvant in the FLUAD™ influenza virus trivalent subunit vaccine.

Particularly preferred adjuvants for use in the compositions are submicron oil-in-water emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80™ (polyoxyelthylenesorbitan monooleate), and/or 0.25-1.0% Span 85™ (sorbitan trioleate), and, optionally, N-acetylmuramyl-L-alanyl-D-isogluatminyl-L-alanine-2-(1'-2'-dipalmitoyl-*sn*-glycero-3-huydroxyphosphophoryloxy)-ethylamine (MTP-PE), for example, the submicron oil-in-water emulsion known as "MF59" (International Publication No. WO90/14837 US Patent Nos. 6, 299, 884 and 6, 451, 325, and Ott et al., "MF59 -- Design and Evaluation of a Safe and Potent Adjuvant for Human Vaccines" in Vaccine Design: The Subunit and Adjuvant Approach (Powell, M.F. and Newman, M.J. eds.) Plenum Press, New York, 1995, pp. 277-296). MF59 contains 4-5% w/v Squalene (e.g. 4.3%), 0.25-0.5% w/v Tween 80™, and 0.5% w/v Span 85™ and optionally contains various amounts of MTP-PE, formulated into submicron particles using a microfluidizer such as Model 110Y microfluidizer (Microfluidics, Newton, MA). For example, MTP-PE may be present in an amount of about 0-500 µg/dose, more preferably 0-250 µg/dose and most preferably, 0-100 µg/dose. As used herein, the term "MF59-0" refers to the above submicron oil-in-water emulsion lacking MTP-PE, while the term MF59-MTP denotes a formulation that contains MTP-PE. For instance, "MF59-100" contains 100 µg MTP-PE per dose, and so on. MF69, another submicron oil-in-water emulsion for use herein, contains 4.3% w/v squalene, 0.25% w/v Tween 80™, and 0.75% w/v Span 85™ and optionally MTP-PE. Yet another submicron oil-in-water emulsion is MF75, also known as SAF, containing 10% squalene, 0.4% Tween 80™, 5% pluronic-blocked polymer L121, and thr-MDP, also microfluidized into a submicron emulsion. MF75-MTP denotes an MF75 formulation that includes MTP, such as from 100-400 µg MTP-PE per dose.

Submicron oil-in-water emulsions, methods of making the same and immunostimulating agents, such as muramyl peptides, for use in the compositions, are described in detail in International Publication No. WO90/14837 and US Patent Nos. 6,299,884 and 6,45 1,325.

Complete Freund's adjuvant (CFA) and incomplete Freund's adjuvant (IFA) may also be used as adjuvants in the invention.

### C. Saponin Formulations

Saponin formulations, may also be used as adjuvants in the invention. Saponins are a heterologous group of sterol glycosides and triterpenoid glycosides that are found in the bark, leaves, stems, roots and even flowers of a wide range of plant species. Saponins isolated from the bark of the *Quillaia saponaria* Molina tree have been widely studied as adjuvants. Saponins can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs.

Saponin compositions have been purified using High Performance Thin Layer Chromatography (HP-TLC) and Reversed Phase High Performance Liquid Chromatography (RP-HPLC). Specific purified fractions using these techniques have been identified, including QS7, QS17, QS18, QS21, QH-A, QH-B and QH-C. Preferably, the saponin is QS21. A method of production of QS21 is disclosed in US Patent No. 5,057,540. Saponin formulations may also comprise a sterol, such as cholesterol (see WO96/33739).

Combinations of saponins and cholesterols can be used to form unique particles called Immunostimulating Complexes (ISCOMs). ISCOMs typically also include a phospholipid such as phosphatidylethanolamine or phosphatidylcholine. Any known saponin can be used in ISCOMs. Preferably, the ISCOM includes one or more of Quil A, QHA and QHC. ISCOMs are further described in EP0109942, WO96/1171 and WO96/33739. Optionally, the ISCOMS may be devoid of (an) additional detergent(s). See WO00/07621. A review of the development of saponin based adjuvants can be found in Barr, et al., "ISCOMs and other saponin based adjuvants", Advanced Drug Delivery Reviews (1998) 32:247-271. See also Sjolander, et al., "Uptake and adjuvant activity of orally delivered saponin and ISCOM vaccines", Advanced Drug Delivery Reviews (1998) 32:321-338.

### D. Virosomes and Virus Like Particle (VLPs)

Virosomes and Virus Like Particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in WO03/024480, WO03/024481, and Niikura et al., "Chimeric Recombinant Hepatitis E Virus-Like Particles as an Oral Vaccine Vehicle Presenting Foreign Epitopes", Virology (2002) 293:273-280; Lenz et al., "Papillomarivurs-Like Particles Induce Acute Activation of Dendritic Cells", Journal of Immunology (2001) 5246-5355; Pinto, et al., "Cellular Immune Responses to Human Papillomavirus (HPV)-16 L1 Healthy Volunteers Immunized with Recombinant HPV-16 L1 Virus-Like Particles", Journal of Infectious Diseases (2003) 188:327-338; and Gerber et al., "Human Papillomavrisu Virus-Like Particles Are Efficient Oral Immunogens when Coadministered with Escherichia coli Heat-Labile Entertoxin Mutant R192G or CpG", Journal of Virology (2001) 75 (10):4752-4760. Virosomes are discussed further in, for example, Gluck et al., "New Technology Platforms in the Development of Vaccines for the Future", Vaccine (2002) 20:B10 -B16. Immunopotentiating reconstituted influenza virosomes (IRIV) are used as the subunit antigen delivery system in the intranasal trivalent INFLEXAL™ product {Mischler & Metcalfe (2002) Vaccine 20 Suppl 5:B17-23} and the INFLUVAC PLUS™ product.

### E. Bacterial or Microbial Derivatives

Adjuvants suitable for use in the invention include bacterial or microbial derivatives such as:

### (1) Non-toxic derivatives of enterobacterial lipopolysaccharide (LPS)

Such derivatives include Monophosphoryl lipid A (MPL) and 3-O-deacylated MPL (3dMPL). 3dMPL is a mixture of 3 De-O-acylated monophosphoryl lipid A with 4, 5 or 6 acylated chains. A preferred "small particle" form of 3 De-O-acylated monophosphoryl lipid A is disclosed in EP 0 689 454. Such "small particles" of 3dMPL are small enough to be sterile filtered through a 0.22 micron membrane (see EP 0 689 454). Other non-toxic LPS derivatives include monophosphoryl lipid A mimics, such as aminoalkyl glucosaminide phosphate derivatives *e.g.* RC-529. See Johnson et al. (1999) Bioorg Med Chem Lett 9:2273-2278.

### (2) Lipid A Derivatives

Lipid A derivatives include derivatives of lipid A from *Escherichia coli* such as OM-174. OM-174 is described for example in Meraldi et al., "OM-174, a New Adjuvant with a Potential for Human Use, Induces a Protective Response with Administered with the Synthetic C-Terminal Fragment 242-310 from the circumsporozoite protein of Plasmodium berghei", Vaccine (2003) 21:2485-2491; and Pajak, et al., "The Adjuvant OM-174 induces both the migration and maturation of murine dendritic cells in vivo", Vaccine (2003) 21:836-842.

### (3) Immunostimulatory oligonucleotides

Immunostimulatory oligonucleotides suitable for use as adjuvants in the invention include nucleotide sequences containing a CpG motif (a sequence containing an unmethylated cytosine followed by guanosine and linked by a phosphate bond). Bacterial double stranded RNA or oligonucleotides containing palindromic or poly(dG) sequences have also been shown to be immunostimulatory.

The CpG's can include nucleotide modifications/analogs such as phosphorothioate modifications and can be double-stranded or single-stranded. Optionally, the guanosine may be replaced with an analog such as 2'-deoxy-7-deazaguanosine. See Kandimalla, et al., "Divergent synthetic nucleotide motif recognition pattern: design and development of potent immunomodulatory oligodeoxyribonucleotide agents with distinct cytokine induction profiles", Nucleic Acids Research (2003) 31(9): 2393-2400; WO02/26757 and WO99/6292 for examples of possible analog substitutions. The adjuvant effect of CpG oligonucleotides is further discussed in Krieg, "CpG motifs: the active ingredient in bacterial extracts?", Nature Medicine (2003) 9(7): 831-835; McCluskie, et al., "Parenteral and mucosal prime-boost immunization strategies in mice with hepatitis B surface antigen and CpG DNA", FEMS Immunology and Medial Microbiology (2002) 32:179-185; WO98/40100; US Patent No. 6,207,646; US Patent No. 6,239,116 and US Patent No. 6,429,199.

The CpG sequence may be directed to TLR9, such as the motif GTCGTT or TTCGTT. See Kandimalla, et al., "Toll-like receptor 9: modulation of recognition and cytokine induction by novel synthetic CpG DNAs", Biochemical Society Transactions (2003) 31 (part 3): 654-658. The CpG sequence may be specific for inducing a Th1 immune response, such as a CpG-A ODN, or it may be more specific for inducing a B cell response, such a CpG-B ODN. CpG-A and CpG-B ODNs are discussed in Blackwell, et al., "CpG-A-Induced Monocyte IFN-gamma-Inducible Protein-10 Production is Regulated by Plasmacytoid Dendritic Cell Derived IFN-alpha", J. Immunol. (2003) 170(8):4061-4068; Krieg, "From A to Z on CpG", TRENDS in Immunology (2002) 23(2): 64-65 and WO01/9593 Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, Kandimalla, et al., "Secondary structures in CpG oligonucleotides affect immunostimulatory activity", BBRC (2003) 306:948-953; Kandimalla, et al., "Toll-like receptor 9: modulation of recognition and cytokine induction by novel synthetic GpG DNAs", Biochemical Society Transactions (2003) 31(part 3):664-658; Bhagat et al., "CpG penta- and hexadeoxyribonucleotides as potent immunomodulatory agents" BBRC (2003) 300:853-861 and WO03/035836.

### (4) ADP-ribosylating toxins and detoxified derivatives thereof.

Bacterial ADP-ribosylating toxins and detoxified derivatives thereof may be used as adjuvants in the invention. Preferably, the protein is derived from *E*. *coli* (i.e., E. coli heat labile enterotoxin "LT), cholera ("CT"), or pertussis ("PT"). The use of detoxified ADP-ribosylating toxins as mucosal adjuvants is described in WO95/17211 and as parenteral adjuvants in WO98/42375. Preferably, the adjuvant is a detoxified LT mutant such as LT-K63, LT-R72, and LTR192G. The use of ADP-ribosylating toxins and detoxified derivatives thereof, particularly LT-K63 and LT-R72, as adjuvants can be found in the following references: Beignon, et al., "The LTR72 Mutant of Heat-Labile Enterotoxin of Escherichia coli Enahnces the Ability of Peptide Antigens to Elicit CD4+ T Cells and Secrete Gamma Interferon after Coapplication onto Bare Skin", Infection and Immunity (2002) 70(6):3012-3019; Pizza, et al., "Mucosal vaccines: non toxic derivatives of LT and CT as mucosal adjuvants", Vaccine (2001) 19:2534-2541; Pizza, et al., "LTK63 and LTR72, two mucosal adjuvants ready for clinical trials" Int. J. Med. Microbiol (2000) 290(4-5):455-461; Scharton-Kersten et al., "Transcutaneous Immunization with Bacterial ADP-Ribosylating Exotoxins, Subunits and Unrelated-Adjuvants", Infection and Immunity (2000) 68(9):5306-5313; Ryan et al., "Mutants of Escherichia coli Heat-Labile Toxin Act as Effective Mucosal Adjuvants for Nasal Delivery of an Acellular Pertussis Vaccine: Differential Effects of the Nontoxic AB Complex and Enzyme Activity on Th1 and Th2 Cells" Infection and Immunity (1999) 67(12):6270-6280: Partidos et al., "Heat-labile enterotoxin of Escherichia coli and its site-directed mutant LTK63 enhance the proliferative and cytotoxic T-cell responses to intranasally co-immunized synthetic peptides", Immunol. Lett. (1999) 67 (3) :209-216; Peppoloni et al., "Mutants of the Escherichia coli heat-labile enterotoxin as safe and strong adjuvants for intranasal delivery of vaccines", Vaccines (2003) 2(2):285-293; and Pine et al., (2002) "Intranasal immunization with influenza vaccine and a detoxified mutant of heat labile enterotoxin from Escherichia coli (LTK63)" J. Control Release (2002) 85(1-3):263-270. Numerical reference for amino acid substitutions is preferably based on the alignments of the A and B subunits of ADP-ribosylating toxins set forth in Domenighini et al., Mol. Microbiol (1995) 15(6):1165-1167.

### F. Bioadhesives and Mucoadhesives

Bioadhesives and mucoadhesives may also be used as adjuvants in the invention. Suitable bioadhesives include esterified hyaluronic acid microspheres (Singh et al. (2001) J. Cont. Rele. 70:267-276) or mucoadhesives such as cross-linked derivatives of polyacrylic acid, polyvinyl alcohol, polyvinyl pyrollidone, polysaccharides and carboxymethylcellulose. Chitosan and derivatives thereof may also be used as adjuvants in the invention. E.g. WO99/27960.

### G. Microparticles

Microparticles may also be used as adjuvants in the invention. Microparticles (*i.e*. a particle of ~100nm to ~150µm in diameter, more preferably -200nm to ~30µm in diameter, and most preferably ~500nm to ~10µm in diameter) formed from materials that are biodegradable and non-toxic (*e.g.* a poly(α-hydroxy acid), a polyhydroxybutyric acid, a polyorthoester, a polyanhydride, a polycaprolactone, *etc*.), with poly(lactide-co-glycolide) are preferred, optionally treated to have a negatively-charged surface (*e.g*. with SDS) or a positively-charged surface (*e.g*. with a cationic detergent, such as CTAB).

### H. Liposomes

Examples of liposome formulations suitable for use as adjuvants are described in US Patent No. 6, 090, 406, US Patent No. 5,916,588, and EP 0 626 169.

### I. Polyoxyethylene ether and Polyoxyethylene Ester Formulations

Adjuvants suitable for use in the invention include polyoxyethylene ethers and polyoxyethylene esters. WO99/52549. Such formulations further include polyoxyethylene sorbitan ester surfactants in combination with an octoxynol (WO01/21207) as well as polyoxyethylene alkyl ethers or ester surfactants in combination with at least one additional non-ionic surfactant such as an octoxynol (WO01/21152).

Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### J. Polyphosphazene (PCPP)

PCPP formulations are described, for example, in Andrianov et al., "Preparation of hydrogel microspheres by coacervation of aqueous polyphophazene solutions", Biomaterials (1998) 19(1-3):109-115 and Payne et al., "Protein Release from Polyphosphazene Matrices", Adv. Drug. Delivery Review (1998) 31(3):185-196.

### K. Muramyl peptides

Examples of muramyl peptides suitable for use as adjuvants in the invention include N-acetyl-muramyl-L-threonyl-D-isoglutamine (thr-MDP), N-acetyl-normuramyl-1-alanyl-d-isoglutamine (nor-MDP), and N-acetylmuramyl-1-alanyl-d-isoglutaminyl-1-alanine-2-(1'-2'-dipalmitoyl-sn-glycero-3-hydroxyphosphoryloxy)-ethylamine MTP-PE).

### L. Imidazoquinoline Compounds.

Examples of imidazoquinoline compounds suitable for use adjuvants in the invention include Imiquimod and its analogues, described further in Stanley, "Imiquimod and the imidazoquinolines: mechanism of action and therapeutic potential" Clin Exp Dermatol (2002) 27(7):571-577; Jones, "Resiquimod 3M", Curr Opin Investig Drugs (2003) 4(2):214-218; and U.S. Patent Nos. 4,689,338, 5,389,640, 5,268,376, 4,929,624, 5,266,575, 5,352,784, 5,494,916, 5,482,936, 5,346,905, 5,395,937, 5,238,944, and 5,525,612.

### M. Thiosemicarbazone Compounds.

Examples of thiosemicarbazone compounds, as well as methods of formulating, manufacturing, and screening for compounds all suitable for use as adjuvants in the invention include those described in WO04/60308. The thiosemicarbazones are particularly effective in the stimulation of human peripheral blood mononuclear cells for the production of cytokines, such as TNF- α.

### N. Tryptanthrin Compounds.

Examples of tryptanthrin compounds, as well as methods of formulating, manufacturing, and screening for compounds all suitable for use as adjuvants in the invention include those described in WO04/64759. The tryptanthrin compounds are particularly effective in the stimulation of human peripheral blood mononuclear cells for the production of cytokines, such as TNF- α.

The invention may also comprise combinations of aspects of one or more of the adjuvants identified above. For example, the following adjuvant compositions may be used in the invention:
(1) a saponin and an oil-in-water emulsion (WO99/11241)
(2) a saponin (e.g.., QS21) + a non-toxic LPS derivative (e.g. 3dMPL) (see WO94/00153);
(3) a saponin (e.g.., QS21) + a non-toxic LPS derivative (e.g. 3dMPL) + a cholesterol;
(4) a saponin (*e.g*. QS21) + 3dMPL + IL-12 (optionally + a sterol) (WO98/57659);
(5) combinations of 3dMPL with, for example, QS21 and/or oil-in-water emulsions (See European patent applications 0835318, 0735898 and 0761231);
(6) SAF, containing 10% Squalane, 0.4% Tween 80, 5% pluronic-block polymer L121, and thr-MDP, either microfluidized into a submicron emulsion or vortexed to generate a larger particle size emulsion.
(7) Ribi™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and
(8) one or more mineral salts (such as an aluminum salt) + a non-toxic derivative of LPS (such as 3dPML).
(9) one or more mineral salts (such as an aluminum salt) + an immunostimulatory oligonucleotide (such as a nucleotide sequence including a CpG motif).

### O. Human Immunomodulators

Human immunomodulators suitable for use as adjuvants in the invention include cytokines, such as interleukins (*e.g*. IL-1, IL-2, IL-4, IL-5, IL-6, IL-7, IL-12, *etc*.), interferons (*e.g*. interferon-γ), macrophage colony stimulating factor, and tumor necrosis factor.

Aluminum salts and MF59 are preferred adjuvants for use with injectable influenza vaccines. Bacterial toxins and bioadhesives are preferred adjuvants for use with mucosally-delivered vaccines, such as nasal vaccines.

The combination vaccines of present invention can be prepared readily according to different methods, all of which are known in the art. Processes and means for the production vaccines are described, for example, in "Vaccines", Plotkin and Orenstein (eds.), 2004. Further, the "Impfcodex, Impfung für Kinder, Erwachsende und Reisende", 5^{th} edition, Chiron Behring GmbH & Co (ed.), 2001 provides useful information. The components of the vaccine can, for example, be mixed before adsorbing the mixture to an appropriate adjuvant. However, as will be appreciated by the those skilled in art, each antigen component can also be adsorbed separately to an adjuvant prior to mixing this component with the other antigen components.

If, for example, the combination vaccine Td-IPV-TBE should be formulated, the individually concentrated IPV antigens of serotype 1, 2, and 3 can be added either to the premixed Td-TBE combination or to the premixed Td combination. In the latter case, the TBE concentrate is added at last. Alternatively, the three individually IPV antigens are mixed to a three-valent IPV concentrate, which is then added to the Td- or Td-TBE premixture. The manufacturing process is similar, if pertussis antigens compounds are to be added in order to create a Td-aP-TBE or a Td-aP-IPV-TBE vaccine. The pertussis antigen containing vaccine may comprise a whole cell fraction, an acellular product or a recombinantly produced product. Preferably, the pertussis component is the pertussis toxoid or a fragment thereof, the filamenteous haemaglutinin antigen, or a protein of the outer membrane.

According to a further embodiment, the different antigen formulations are blended before bottling the combination vaccine. This means, the different antigen formulations can be mixed to a combined multivalent vaccine, and subsequently absorbed to a suitable carrier/adjuvant, such as aluminum hydroxide.

The production process has to be performed in accordance with the guidelines of good laboratory practice. For example, the mixing of different antigen formulations has to be performed under sterile conditions so that the resulting vaccine is not contaminated with hazardous substances such as infective agents which would be harmful to the immunized subject. Generally, mixing and bottling of the combination vaccine can be performed according to methods well known in the art.

According to a further aspect, the invention relates to a kit comprising at least one antigen conferring protection against tetanus, at least one antigen conferring protection against diphtheria; and at least one antigen conferring protection against tick-borne encephalitis and in addition further reagents which are suitable for preparing a combination vaccine according to the invention. Preferably, the kits of the invention comprise at least one antigen of Clostridium tetani, at least one antigen of Corynebacterium diphtheriae, and at least one antigen of a TBE-virus. Additional reagents can comprise, for example, buffers, stabilizing agents, anti-oxidants and/or preservatives.

The invention also provides kits comprising one or more containers of compositions of the invention. Compositions can be in liquid form or can be lyophilized, as can individual antigens. Suitable containers for the compositions include, for example, bottles, vials, syringes, and test tubes. Containers can be formed from a variety of materials, including glass or plastic. A container may have a sterile access port (for example, the container may be an intravenous solution bag or a vial having a stopper pierceable by a hypodermic injection needle).

The kit can further comprise a second container comprising a pharmaceutically-acceptable buffer, such as phosphate-buffered saline, Ringer's solution, or dextrose solution. It can also contain other materials useful to the end-user, including other pharmaceutically acceptable formulating solutions such as buffers, diluents, filters, needles, and syringes or other delivery device. The kit may further include a third component comprising an adjuvant.

The kit can also comprise a package insert containing written instructions for methods of inducing immunity or for treating infections. The package insert can be an unapproved draft package insert or can be a package insert approved by the Food and Drug Administration (FDA) or other regulatory body.

The invention also provides a delivery device pre-filled with the immunogenic compositions of the invention.

Preferably, the antigens of the combination vaccine are stored separately for instantaneous mixing before injection. Here, the antigens can be present in separate vials, either as a solution or in lyophilized form for reconstitution. According to a further embodiment, the antigen formulations can be present in a syringe with two or more separate chambers. Upon injection, these chambers are joined so that the different antigen formulations mix up shortly before injection to the combination vaccine of the present invention.

According to a further aspect, the invention relates to a method for preparing a combination according to the invention, in which at least one tetanus toxoid conferring protection against tetanus, at least one diphtheria toxoid conferring protection against diphtheria, and at least one antigen from TBE virus strain Neudörfl or TBE virus strain K23 conferring protection against tick-borne encephalitis are mixed and the mixed antigens are subsequently absorbed to a suitable adjuvant. Alternatively, the invention relates to a method for preparing a combination according to the invention, in which at least one tetanus toxoid conferring protection against tetanus, at least one diphtheria toxoid conferring protection against diphtheria, and at least one antigen from TBE virus strain Neudörfl or TBE virus strain K23 conferring protection against tick-borne encephalitis are absorbed separately from each other to a suitable adjuvant and the adsorbed antigens are subsequently mixed with each other.

The invention also provides a composition of the invention for use as a medicament. The medicament is preferably able to raise an immune response in a mammal (i.e. it is an immunogenic composition) . The invention also provides the use of the compositions of the invention in the manufacture of a medicament for raising an immune response in a mammal.

The mammal is preferably a human. Preferably the vaccine is for prophylactic use, the human is preferably a child (e.g. a toddler or infant, preferably pre-school, preferably one year or less or from three years onwards) or a teenager; where the vaccine is for therapeutic use, the human is preferably a teenager or an adult. A vaccine intended for children may also be administered to adults *e.g.* to assess safety, dosage, immunogenicity, *etc*. Preferably, the human is a teenager. More preferably, the human is a pre-adolescent teenager. Even more preferably, the human is a pre-adolescent female or male. Preferably the pre-adolescent male or female is around 9-12 years of age. Preferably the adolescent male or female is around 15-19 years of age. Preferably the male or female is around 20-49 years of age. Preferably the male or female is over 49 years of age.

Compositions of the invention will generally be for administration directly to a patient. Direct delivery may be accomplished by parenteral injection (*e.g*. subcutaneously, intraperitoneally, intravenously, intramuscularly, or to the interstitial space of a tissue), or mucosally, such as by rectal, oral (*e.g*. tablet, spray), vaginal, topical, transdermal (See *e.g.* WO99/27961) or transcutaneous (See *e.g.* WO02/074244 and WO02/064162), intranasal (See *e.g*. WO03/028760), ocular, aural, pulmonary or other mucosal administration.

The immunogenic composition may be for use in eliciting systemic and/or mucosal immunity, preferably to elicit an enhanced systemic and/or mucosal immunity.

Dosage treatment can be a single dose schedule or a multiple dose schedule. Multiple doses may be used in a primary immunisation schedule and/or in a booster immunisation schedule. In a multiple dose schedule the various doses may be given by the same or different routes *e.g*. a parenteral prime and mucosal boost, a mucosal prime and parenteral boost, *etc*.

Preferably the immunogenic composition is administered using a rapid immunisation schedule comprising three immunisations at days 0, 7 and 21 generally followed by a booster dose at months 12-18 if long term protection is required (see Zent et al (2004) Vaccine 23; 312-315 and Zent et al (2005) J Travel Med 17: 331-342). This rapid immunisation schedule where the three doses for completion of a primary immunisation are given within three weeks contrasts with a conventional schedule in which the three doses required for primary immunisation are given within one year on Day 0, after 1-3 months and 9-12 months. The rapid immunisation schedule is quite similar to schedules for other vaccines like Rabies pre-exposure vaccination and have been evaluated for other viral vaccines, such as Hepatitis A and B and Japan encephalitis vaccines (Nothdurft et al (2002) Vaccine 20: 1157-1162). It is especially suitable in situations, where the convention schedule cannot be followed due to time restraints, for example, for people living in endemic areas during tick season and for travellers shortly before travelling to endemic areas. Especially for the latter, booster recommendations might not always be followed.

Preferably the dosage regime enhances the avidity of the antibody response leading to antibodies with a neutralising characteristic.

### Tests to determine the efficacy of the immune response

One way of assessing efficacy of therapeutic treatment involves monitoring infection after administration of the composition of the invention. One way of assessing efficacy of prophylactic treatment involves monitoring immune responses against the antigens in the compositions of the invention after administration of the composition.

Another way of assessing the immunogenicity of the component proteins of the immunogenic compositions of the present invention is to express the proteins recombinantly and to screen patient sera or mucosal secretions by immunoblot. A positive reaction between the protein and the patient serum indicates that the patient has previously mounted an immune response to the protein in question- that is, the protein is an immunogen. This method may also be used to identify immunodominant proteins and/or epitopes.

As used herein, the term "epitope" generally refers to the site on an antigen which is recognised by a T-cell receptor and/or an antibody. Preferably it is a short peptide derived from or as part of a protein antigen. However the term is also intended to include peptides with glycopeptides and carbohydrate epitopes. Several different epitopes may be carried by a single antigenic molecule. The term "epitope" also includes modified sequences of amino acids or carbohydrates which stimulate responses which recognise the whole organism. It is advantageous if the selected epitope is an epitope of an infectious agent, which causes the infectious disease.

The epitope can be generated from knowledge of the amino acid and corresponding DNA sequences of the peptide or polypeptide, as well as from the nature of particular amino acids (e.g., size, charge, etc.) and the codon dictionary, without undue experimentation. See, e.g., Ivan Roitt, Essential Immunology, 1988; Kendrew, supra; Janis Kuby, Immunology, 1992 e.g., pp. 79-81. Some guidelines in determining whether a protein will stimulate a response, include: Peptide length-preferably the peptide is about 8 or 9 amino acids long to fit into the MHC class I complex and about 13-25 amino acids long to fit into a class II MHC complex. This length is a minimum for the peptide to bind to the MHC complex. It is preferred for the peptides to be longer than these lengths because cells may cut peptides. The peptide may contain an appropriate anchor motif which will enable it to bind to the various class I or class II molecules with high enough specificity to generate an immune response (See Bocchia, M. et al, Specific Binding of Leukemia Oncogene Fusion Protein Pentides to HLA Class I Molecules, Blood 85:2680-2684; Englehard, VH, Structure of peptides associated with class I and class II MHC molecules Ann. Rev. Immunol. 12:181 (1994)). This can be done, without undue experimentation, by comparing the sequence of the protein of interest with published structures of peptides associated with the MHC molecules. Thus, the skilled artisan can ascertain an epitope of interest by comparing the protein sequence with sequences listed in the protein database.

As used herein, the term "T cell epitope" refers generally to those features of a peptide structure which are capable of inducing a T cell response and a "B cell epitope" refers generally to those features of a peptide structure which are capable of inducing a B cell response.

Another way of checking efficacy of therapeutic treatment involves monitoring infection after administration of the compositions of the invention. One way of checking efficacy of prophylactic treatment involves monitoring immune responses both systemically (such as monitoring the level of IgG1 and IgG2a production) and mucosally (such as monitoring the level of IgA production) against the antigens in the compositions of the invention after administration of the composition. Typically, serum specific antibody responses are determined post-immunization but pre-challenge whereas mucosal specific antibody body responses are determined post-immunization and post-challenge.

The immunogenic compositions of the present invention can be evaluated in *in vitro* and *in vivo* animal models prior to host, *e.g*., human, administration. Particularly useful mouse models include those in which intraperitoneal immunization is followed by either intraperitoneal challenge or intranasal challenge.

The efficacy of immunogenic compositions of the invention can also be determined *in vivo* by challenging animal models of infection, *e.g*., guinea pigs or mice, with the immunogenic compositions. The immunogenic compositions may or may not be derived from the same strains as the challenge strains. Preferably the immunogenic compositions are derivable from the same strains as the challenge strains.

*In vivo* efficacy models include but are not limited to: (i) A murine infection model using human strains; (ii) a murine disease model which is a murine model using a mouse-adapted strain, such as strains which are particularly virulent in mice and (iii) a primate model using human isolates.

The immune response may be one or both of a TH1 immune response and a TH2 response.

The immune response may be an improved or an enhanced or an altered immune response.

The immune response may be one or both of a systemic and a mucosal immune response.

Preferably the immune response is an enhanced system and/or mucosal response.

An enhanced systemic and/or mucosal immunity is reflected in an enhanced TH1 and/or TH2 immune response. Preferably, the enhanced immune response includes an increase in the production of IgG1 and/or IgG2a and/or IgA

Preferably the mucosal immune response is a TH2 immune response. Preferably, the mucosal immune response includes an increase in the production of IgA.

Activated TH2 cells enhance antibody production and are therefore of value in responding to extracellular infections. Activated TH2 cells may secrete one or more of IL-4, IL-5, IL-6, and IL-10. A TH2 immune response may result in the production of IgG1, IgE, IgA and memory B cells for future protection.

A TH2 immune response may include one or more of an increase in one or more of the cytokines associated with a TH2 immune response (such as IL-4, IL-5, IL-6 and IL-10), or an increase in the production of IgG1, IgE, IgA and memory B cells. Preferably, the enhanced TH2 immune response will include an increase in IgG1 production.

A TH1 immune response may include one or more of an increase in CTLs, an increase in one or more of the cytokines associated with a TH1 immune response (such as IL-2, IFNγ, and TNFβ), an increase in activated macrophages, an increase in NK activity, or an increase in the production of IgG2a. Preferably, the enhanced TH1 immune response will include an increase in IgG2a production.

Immunogenic compositions of the invention, in particular, immunogenic composition comprising one or more antigens of the present invention may be used either alone or in combination with other antigens optionally with an immunoregulatory agent capable of eliciting a Th1 and/or Th2 response.

The invention also comprises an immunogenic composition comprising one or more immunoregulatory agent, such as a mineral salt, such as an aluminium salt and an oligonucleotide containing a CpG motif. Most preferably, the immunogenic composition includes both an aluminium salt and an oligonucleotide containing a CpG motif. Alternatively, the immunogenic composition includes an ADP ribosylating toxin, such as a detoxified ADP ribosylating toxin and an oligonucleotide containing a CpG motif. Preferably, the one or more immunoregulatory agents include an adjuvant. The adjuvant may be selected from one or more of the group consisting of a TH1 adjuvant and TH2 adjuvant, further discussed above.

The immunogenic compositions of the invention will preferably elicit both a cell mediated immune response as well as a humoral immune response in order to effectively address an infection. This immune response will preferably induce long lasting (*e.g*., neutralizing) antibodies and a cell mediated immunity that can quickly respond upon exposure to one or more infectious antigens. By way of example, evidence of neutralizing antibodies in patients blood samples is considered as a surrogate parameter for protection since their formation is of decisive importance for virus elimination in TBE infections (see Kaiser and Holzmann (2000) Infection 28; 78-84).

The invention will be further illustrated by the following non-limiting following examples.

### Examples

### Example 1: Production of a Td-TBE combination vaccine

This example discloses manufacturing of a Td-TBE combination vaccine. Preferentially, all the antigens are individually adsorbed to aluminium hydroxide for at least 30 minutes.

Tetanus and diphtheria toxoids are obtained and purified according to methods well known in the art. Briefly, common semi-synthetic media are inoculated with Clostridium tetani (Harvard strain) after 1 to 2 passages as a pre-culture into a fermenter. The cells are cultured for 6 to 7 days. Tetanus toxin is produced within the first three days after inoculation. The toxin is released in the medium after lysis of the cells. At the end of the culturing phase, the toxin is obtained by sterile filtration and detoxified by the addition of formalin and incubation at 37° C for 3-4 weeks. Toxoid concentrate is obtained by ultrafiltration and salt precipitation. Similarly, cultures of Corynebacterium diphtheriae (strain Park & Williams, BW 8) is inoculated into a fermenter after 1-2 pre-culture passages. The cells are cultured for 48 hours under stirring and strong aeration. The culturing temperature usually is 33°-37°C. Diphtheria toxin is secreted by cell into the culture supernatant. The toxin is obtained by sterile filtration and is usually incubated (without the addition of formalin) for several weeks. Subsequently, formalin is added and the toxin is detoxified for six weeks by incubation at 37°C. Purification and concentration is performed by ultrafiltration and ammoniumsulphate precipitation.

Alternatively, one can also use ready-to-use combination vaccines such as Td-pur® (Chiron Behring GmbH & Co KG) or DT Impfstoff für Kinder (Chiron Behring GmbH & Co KG) as a starting material. Briefly, Td-pur® comprises about 20 IU/dose tetanus toxoid (which corresponds to about 20 Lf) and about 2 IU/dose diphtheria toxoid (which corresponds to about 1.5 Lf). In comparison, the DT Impfstoff für Kinder contains at least 40 IU/dose tetanus toxoid (which corresponds to about 20 Lf) and at least 30 IU/dose diphtheria toxoid (which corresponds to about 20 Lf). In each case, a concentration of 15 g/l aluminium hydroxide is used.

In order to prepare the vaccine, tetanus antigen is adsorbed to the aluminium hydroxide to give a concentrate of 750 Lf/ml. Similarly, the diphtheria toxoid is adsorbed to give a concentrate of 300 Lf/ml. Finally, purified TBE-whole virus fraction is used to give a concentrate of 60 µg/ml. The tetanus and diphtheria toxoid suspensions are 75 fold concentrated compared to the concentration of the final combination vaccine, and the TBE antigen concentration is 20 fold.

The concentration of aluminium hydroxide is 15 g/l for each concentrate. These concentrates are transferred to a formulation vessel (Hermann Waldner GmbH & Co KG, Wangen, Germany or Pharmatec Deutschland GmbH, Dresden, Germany) and then an appropriate amount of aluminium hydroxide suspension (15 g/l is added to give a final concentration of 2.0 mg aluminium hydroxide/ml in the final combination vaccine. Then, sterile filtrated saccharose solution is added to give a final concentration of 50 mg/ml and sterile filtrated NaCl solution and water for injection is added to fill the master batch to the final volume. If necessary, the pH value is adjusted to 6.8-7.8. and the master batch is agitated for 30 minutes. Then, the TBE-Td vaccine portions were bottled into vials or syringes. Several further combination vaccines can be produced according to this process, inter alia, Td-TBE-aP, Td-TBE-IPV and Td-TBE-aP-IPV combinations.

### Example 2: Modes of administration and storing

Different modes of storing/administration may be used in view of putative differences with respect to the effectiveness of the combination vaccine. Briefly, the following approaches may be used:
A. Td and TBE antigens are combined in a two-chamber vaccine syringe (Becton-Dickinson) or two vials, such that the two liquid components are stored separately in the device, and the antigens are instantaneously at the time of injection.
B. One of the two antigen components (either the Td or the TBE component) is lyophilised by use of a lyophilizer (Hof-Sonderanlagenbau, Lohra, Germany), while the other antigen is obtained in liquid form from the process described in example 1. The lyophilised component is reconstituted by the liquid component prior to the immunization.
C. The Td and the TBE antigens are blended in one suspension in a syringe or a vial and the mixture is stored for 7 days at 4°C prior to the immunization.

### Example 3: Production of a tetravalent or pentavalent Td-TBE combination vaccines

As described in Example 1, all components are separately adsorbed to aluminium hydroxide and subsequently mixed.

Instead of using Td for blending with the TBE component according to the above mentioned examples, a trivalent component like Td-aP (Td together with acellular pertussis antigen; obtainable, for example, as monovalent vaccine Acelluvax of Chiron Vaccines, Chiron S.p.A., Siena, Italy or Td-IPV (inactivated polio virus; antigen obtainable, for example, from The Netherland Vaccine Institute, Bilthoven, The Netherland) may be used resulting in a tetravalent Td-TBE-aPor Td-TBE-IPV vaccine, respectively. In addition, an even broader combination is possible, e.g. Td-TBE-aP-IPV vaccine.

### EXAMPLE 4: Immunization witch T, D(d), TBE antigen combinations either alone or in combination with an immunoregulatory agent

The following example illustrates immunization with various combinations of T, D(d), TBE antigens in a mouse model. The T, D(d), TBE antigens are prepared and characterized as described herein.
CD1 mice are divided into nine groups and immunized as follows:

**Table: Immunization Schedule for Example 4**

| **Group** | **Immunizing Composition** | **Route of Delivery** |
|---|---|---|
| 1 | Mixture of T, D(d) and TBE antigens (5 µg/each) + CFA | Intra-peritoneal or intranasal |
| 2 | Mixture of T, D(d) and TBE antigens (5 µg/each) +AlOH (200µg) | Intra-peritoneal or intranasal |
| 3 | Mixture of T, D(d) and TBE antigens (5 µg/each) +CpG (10ug) | Intra-peritoneal or intranasal |
| 4 | Mixture of T, D(d) and TBE antigens (5 µg/each) + AlOH (200µg) + CpG (10µg) | Intra-peritoneal or intranasal |
| 5 | Complete Freunds Adjuvant (CFA) | Intra-peritoneal or intranasal |
| 6 | Mixture of T, D(d) and TBE (5 µg/each) + LTK63 (5µg) | Intra-peritoneal or Intranasal |
| 7 | AlOH (200µg) + CpG (10µg) | Intra-peritoneal or intranasal |
| 8 | CpG (10µg) | Intra-peritoneal or intranasal |
| 9 | LTK63 (5µg) | Intra-peritoneal or intranasal |

Mice are immunized at two week intervals. Two weeks after the last immunization, all mice are challenged with the appropriate strain. When mucosal immunization (eg intra-nasal(in)) is used, the animal model is also challenged mucosally to test the protective effect of the mucosal immunogen.

## Claims

1. Combination vaccine comprising
a) a tetanus toxoid conferring protection against tetanus,
b) a diphtheria toxoid conferring protection against diphtheria; and
c) an antigen from TBE-virus strain Neudörfl or TBE-virus strain K23 conferring protection against tick-borne encephalitis.

2. Combination vaccine according to claim 1, wherein said antigen conferring protection against tick-borne encephalitis is the envelope glycoprotein E of the TBE-virus.

3. Combination vaccine according to claims 1 or 2, wherein said antigen conferring protection against tick-borne encephalitis is a whole virus fraction of the TBE-virus.

4. Combination vaccine according to claim 3, wherein said antigen conferring protection against tick-borne encephalitis is present in an amount of about 0.1 to 5 µg/dose, preferably 0.5 to 2.5 µg/dose.

5. Combination vaccine according to claim 4, wherein said antigen conferring protection against tick-borne encephalitis is present in an amount of 0.01 µg/dose to about 10 µg/dose.

6. Combination vaccine according to claim 1 to 5, wherein said tetanus toxoid is derived from Clostridium tetani strain Havard.

7. Combination vaccine according to claim 1 to 6, wherein said tetanus toxoid is present in an amount which corresponds to a potency of about 10-60 IU/dose, preferably 10-50 IU/dose and more preferably at least 20 IU/dose.

8. Combination vaccine according to claims 1 to 7, wherein said diphtheria toxoid is derived from Corynebacterium diphtheriae strain Massachusetts 8 Park Williams.

9. Combination vaccine according to claim 1 to 8, wherein said diphtheria toxoid is present in an amount which corresponds to a potency of about 10-50 IU/dose, preferably 20-40 IU/dose and more preferably at least 30 IU/dose.

10. Combination vaccine according to claim 1 to 9, wherein said diphtheria toxoid is present in an amount which corresponds to a potency of about 0.5-10 IU/dose, preferably 0.75-5 IU/dose and more preferably at least 2 IU/dose.

11. Combination vaccine according to any of claims 1 to 10, wherein said tetanus toxoid is present in an amount which corresponds to a potency of at least 20 IU/dose, said diphtheria toxoid is present in an amount which corresponds to a potency of at least 2 IU/dose, and said antigen conferring protection against tick-borne encephalitis is present in an amount of 0.75 µg/dose.

12. Combination vaccine according to one of the preceding claims, wherein the vaccine further comprises an adjuvant.

13. Combination vaccine according to claim 12, wherein said adjuvant is an aluminium salt, such as aluminium hydroxide.

14. Combination vaccine according to one of the preceding claims, wherein the vaccine further comprises at least one additional antigen from another pathogenic microorganism.

15. Combination vaccine according to claim 14, wherein said at least one additional antigen is capable of conferring protection against a disease or medical condition selected from a group of pertussis, polio, hepatitis A, meningococcal diseases or Lyme disease.

16. Combination vaccine according to claim 15, wherein said at least one additional antigen is capable of conferring protection against pertussis.

17. Use of a tetanus toxoid conferring protection against tetanus, a diphtheria toxoid conferring protection against diphtheria and an antigen from TBE-virus strain Neudörfl or TBE-virus strain K23 conferring protection against tick-borne encephalitis for the preparation of a combination vaccine for the prophylactic protection against tetanus, diphtheria and tick-borne encephalitis.

18. Use according to claim 17, wherein said antigens are antigens of Clostridium tetani, Corynebacterium diphtheriae and from TBE-virus strain Neudörfl or TBE-virus strain K23.

19. Kit comprising a tetanus toxoid conferring protection against tetanus, a diphtheria toxoid conferring protection against diphtheria, an antigen from TBE-virus strain Neudörfl or TBE-virus strain K23 conferring protection against tick-borne encephalitis and reagents suitable for preparing a combination vaccine.

20. Kit according to claim 19, wherein said antigens are antigens of Clostridium tetani, Corynebacterium diphtheriae and from TBE-virus strain Neudörfl or TBE-virus strain K23.

21. Kit according to claims 19 or 20, wherein said antigens are separately stored.

22. Kit according to claim 21, wherein the antigens are present in a syringe with two or more separate chambers.

23. Kit according to claim 21, wherein the antigens are present in two or more separate vials.

24. Method for preparing a combination vaccine of one of claims 1 to 16, comprising the steps of:
a) mixing a tetanus toxoid conferring protection against tetanus, a diphtheria toxoid conferring protection against diphtheria, and an antigen from TBE-virus strain Neudörfl or TBE-virus strain K23 conferring protection against tick-borne encephalitis; and
b) adsorbing the mixed antigens to a suitable adjuvant.

25. Method for preparing a combination vaccine of one of claims 1 to 16, comprising the steps of:
a) adsorbing a tetanus toxoid conferring protection against tetanus, a diphtheria toxoid conferring protection against diphtheria, and an antigen from TBE-virus strain Neudörfl or TBE-virus strain K23 conferring protection against tick-borne encephalitis separately from each other to a suitable adjuvant; and
b) mixing the adsorbed antigens with each other.

## Patentansprüche

1. Kombinationsimpfstoff, umfassend
a) ein Tetanus-Toxoid, das Schutz gegen Tetanus verleiht;
b) ein Diphtherie-Toxoid, das Schutz gegen Diphtherie verleiht; und
c) ein Antigen des TBE-Virusstamms Neudörfl oder des TBE-Virusstamms K23, das Schutz gegen Frühsommer-Meningoenzephalitis verleiht.

2. Kombinationsimpfstoff gemäß Anspruch 1, wobei das Antigen, welches Schutz gegen Frühsommer-Meningoenzephalitis verleiht, das Hüllen-Glycoprotein E des TBE-Virus ist.

3. Kombinationsimpfstoff gemäß Anspruch 1 oder 2, wobei das Antigen, welches Schutz gegen Frühsommer-Meningoenzephalitis verleiht, eine Gesamt-Virus-Fraktion des TBE-Virus ist.

4. Kombinationsimpfstoff gemäß Anspruch 3, wobei das Antigen, welches Schutz gegen Frühsommer-Meningoenzephalitis verleiht, in einer Menge von etwa 0,1 bis 5 µg/Dosis, vorzugsweise 0,5 bis 2,5 µg/Dosis, vorhanden ist.

5. Kombinationsimpfstoff gemäß Anspruch 4, wobei das Antigen, welches Schutz gegen Frühsommer-Meningoenzephalitis verleiht, in einer Menge von 0,01 µg/Dosis bis etwa 10 µg/Dosis vorhanden ist.

6. Kombinationsimpfstoff gemäß einem der Ansprüche 1 bis 5, wobei das Tetanus-Toxoid vom Harvard-Stamm von Clostridium tetani abgeleitet ist.

7. Kombinationsimpfstoff gemäß einem der Ansprüche 1 bis 6, wobei das Tetanus-Toxoid in einer Menge vorhanden ist, die einer Potenz von etwa 10-60 IU/Dosis, vorzugsweise 10-50 IU/Dosis und stärker bevorzugt mindestens 20 IU/Dosis entspricht.

8. Kombinationsimpfstoff gemäß einem der Ansprüche 1 bis 7, wobei das Diphtherie-Toxoid vom Massachusetts 8 Park Williams-Stamm von Corynebacterium diphtheriae abgeleitet ist.

9. Kombinationsimpfstoff nach einem der Ansprüche 1 bis 8, wobei das Diphtherie-Toxoid in einer Menge vorhanden ist, die einer Potenz von etwa 10-50 IU/Dosis, vorzugsweise 20-40 IU/Dosis, und stärker bevorzugt mindestens 30 IU/Dosis entspricht.

10. Kombinationsimpfstoff gemäß einem der Ansprüche 1 bis 9, wobei das Diphtherie-Toxoid in einer Menge vorliegt, die einer Potenz von etwa 0,5-10 IU/Dosis, vorzugsweise 0,75-5 IU/Dosis, und stärker bevorzugt mindestens 2 IU/Dosis entspricht.

11. Kombinationsimpfstoff gemäß einem der Ansprüche 1 bis 10, wobei das Tetanus-Toxoid in einer Menge vorhanden ist, die einer Potenz von mindestens 20 IU/Dosis entspricht, das Diphtherie-Toxoid in einer Menge vorhanden ist, die einer Potenz von mindestens 2 IU/Dosis entspricht und das Antigen, welches Schutz gegen Frühsommer-Meningoenzephalitis verleiht, in einer Menge von 0,75 µg/Dosis vorhanden ist.

12. Kombinationsimpfstoff gemäß einem der vorherigen Ansprüche wobei der Impfstoff ferner ein Adjuvans umfasst.

13. Kombinationsimpfstoff gemäß Anspruch 12, wobei das Adjuvans ein Aluminiumsalz ist, wie beispielsweise Aluminiumhydroxid.

14. Kombinationsimpfstoff gemäß einem der vorherigen Ansprüche, wobei der Impfstoff ferner mindestens ein zusätzliches Antigen von einem anderen pathogenen Mikroorganismus umfasst.

15. Kombinationsimpfstoff gemäß Anspruch 14, wobei das mindestens eine zusätzliche Antigen in der Lage ist, Schutz gegen eine Erkrankung oder einen medizinischen Zustand zu verleihen, die/der ausgewählt ist aus der Gruppe bestehend aus Keuchhusten, Kinderlähmung, Hepatitis A, Meningokokken-Erkrankung oder Lyme-Krankheit.

16. Kombinationsimpfstoff gemäß Anspruch 15, wobei das mindestens eine zusätzliche Antigen in der Lage ist, Schutz gegen Keuchhusten zu verleihen.

17. Verwendung eines Tetanus-Toxoids, das Schutz gegen Tetanus verleiht, eines Diphtherie-Toxoids, das Schutz gegen Diphtherie verleiht, und eines Antigens vom TBE-Virusstamm Neudörfl oder des TBE-Virusstamms K23, das Schutz gegen Frühsommer-Meningoenzephalitis verleiht, zur Herstellung eines Kombinationsimpfstoffes für den prophylaktischen Schutz gegen Tetanus, Diphtherie und Frühsommer-Meningoenzephalitis.

18. Verwendung gemäß Anspruch 17, wobei es sich bei den Antigenen um Antigene von Clostridium tetani, Corynebacterium diphtheriae und solche des TBE-Virusstamms Neudörfl oder des TBE-Virusstamms K23 handelt.

19. Kit, der ein Tetanus-Toxoid umfasst, das Schutz gegen Tetanus verleiht, ein Diphtherie-Toxoid, das Schutz gegen Diphtherie verleiht, ein Antigen des TBE-Virusstamms Neudörfl oder des TBE-Virusstamms K23, das Schutz gegen Frühsommer-Meningoenzephalitis verleiht, und Mittel, die für die Herstellung eines Kombinationsimpfstoffes geeignet sind.

20. Kit gemäß Anspruch 19, wobei es sich bei den Antigenen um Antigene von Clostridium tetani, Corynebacterium diphtheriae und solche des TBE-Virusstamms Neudörfl oder des TBE-Virusstamms K23 handelt.

21. Kit gemäß Anspruch 19 oder 20, wobei die Antigene getrennt voneinander aufbewahrt werden.

22. Kit gemäß Anspruch 21, wobei die Antigene in einer Spritze mit zwei oder mehreren getrennten Kammern vorliegen.

23. Kit gemäß Anspruch 21, wobei die Antigene in zwei oder mehreren getrennten Fläschchen vorliegen.

24. Verfahren zur Herstellung eines Kombinationsimpfstoffes gemäß einem der Ansprüche 1 bis 16, bei dem man:
a) ein Tetanus-Toxoid, das Schutz gegen Tetanus verleiht, ein Diphtherie-Toxoid, das Schutz gegen Diphtherie verleiht, und ein Antigen des TBE-Virusstamms Neudörfl oder des TBE-Virusstamms K23, das Schutz gegen Frühsommer-Meningoenzephalitis verleiht, mischt;
und
b) die gemischten Antigene an ein geeignetes Adjuvans adsorbiert.

25. Verfahren zur Herstellung eines Kombinationsimpfstoffes gemäß einem der Ansprüche 1 bis 16, bei dem man:
a) ein Tetanus-Toxoid, das Schutz gegen Tetanus verleiht, ein Diphtherie-Toxoid, das Schutz gegen Diphtherie verleiht, und ein Antigen des TBE-Virusstamms Neudörfl oder des TBE-Virusstamm K23, das Schutz gegen Frühsommer-Meningoenzephalitis verleiht, getrennt voneinander an ein geeignetes Adjuvans adsorbiert;
und
b) die adsorbierten Antigene miteinander vermischt.

## Revendications

1. Vaccin combiné comprenant
a) un toxoïde de tétanus conférant une protection contre le tétanus,
b) un toxoïde de diphtérie conférant une protection contre la diphtérie ; et
c) un antigène de la souche Neudörfl du virus de TBE ou de la souche K23 du virus de la TBE conférant une protection contre l'encéphalite à tiques.

2. Vaccin combiné selon la revendication 1, dans lequel ledit antigène conférant une protection contre l'encéphalite à tiques est la glycoproptéine d'enveloppe E du virus de la TBE.

3. Vaccin combiné selon les revendications 1 ou 2, dans lequel ledit antigène conférant une protection contre l'encéphalite à tiques est une fraction de virus complète du virus de la TBE.

4. Vaccin combiné selon la revendication 3, dans lequel ledit antigène conférant une protection contre l'encéphalite à tiques est présent en une quantité d'environ 0,1 à 5 µg/dose, de préférence 0,5 à 2,5 µg/dose.

5. Vaccin combiné selon la revendication 4, dans lequel ledit antigène conférant une protection contre l'encéphalite à tiques est présent en une quantité de 0,01 µg/dose à environ 10µg/dose.

6. Vaccin combiné selon la revendication 1 à 5, dans lequel ledit toxoïde de tétanus est dérivé de la souche Havard de Clostridium tétani.

7. Vaccin combiné selon la revendication 1 à 6, dans lequel ledit toxoïde de tétanus est présent en une quantité qui correspond à une puissance d'environ 10-60 IU/dose, de préférence 10-50 IU/dose et selon une plus grande préférence à au moins 20 IU/dose.

8. Vaccin combiné selon les revendications 1 à 7, dans lequel ledit toxoïde de diphtérie est dérivé de la souche Corynebactérium diphtériae, Massachusetts 8 Park Williams.

9. Vaccin combiné selon la revendication 1 à 8, dans lequel ledit toxoïde de diphtérie est présent en une quantité qui correspond à une puissance d'environ 10-50 IU/dose, de préférence 20-40 IU/dose et selon une plus grande préférence à au moins 30 IU/dose.

10. Vaccin combiné selon la revendication 1 à 9, dans lequel ledit toxoïde de diphétérie est présent en une quantité qui correspond à une puissance d'environ 0,5-10 IU/dose, de préférence 0,75-5 IU/dose et selon une plus grande préférence à au moins 2 IU/dose.

11. Vaccin combiné selon l'une quelconque des revendications 1 à 10, dans lequel ledit toxoïde tétanus est présent en une quantité qui correspond à une puissance d'au moins 20 IU/dose, ledit toxoïde de diphtérie est présent en une quantité qui correspond à une puissance d'au moins 2 IU/dose, et ledit antigène conférant une protection contre l'encéphalite à tiques est présent en une quantité de 0,75µg/dose.

12. Vaccin combiné selon l'une quelconque des revendications précédentes, dans lequel le vaccin comprend en outre un adjuvant.

13. Vaccin combiné selon la revendication 12, dans lequel ledit adjuvant est un sel d'aluminium, comme l'hydroxyde d'aluminium.

14. Vaccin combiné selon l'une quelconque des revendications précédentes, dans lequel le vaccin comprend en outre au moins un antigène additionnel d'un autre micro-organisme pathogène.

15. Vaccin combiné selon la revendication 14, dans lequel ledit au moins un antigène additionnel est apte à conférer une protection contre une maladie ou une condition médicale, sélectionnée dans un groupe de coqueluche, polio, hépatite A, méningococcies ou maladie de Lyme.

16. Vaccin combiné selon la revendication 15, dans lequel ledit au moins un antigène additionnel est apte à conférer une protection contre la coqueluche.

17. Utilisation d'un toxoïde de tétanus conférant une protection contre le tétanus, un toxoïde de diphtérie conférant une protection contre la diphtérie et un antigène de la souche Neudörfl du virus de la TBE ou de la couche K23 du virus de la TBE conférant une protection contre l'encéphalite à tiques pour la préparation d'un vaccin combiné pour la protection prophylactique contre le tétanos, la diphtérie et l'encéphalite à tiques.

18. Utilisation selon la revendication 17, où lesdits antigènes sont des antigènes de Clostridium tétani, Corynebactérium diphtériae et de la souche Neudörfl du virus de la TBE ou de la souche K23 du virus de la TBE.

19. Kit comprenant un toxoïde de tétanos conférant une protection contre le tétanos, un toxoïde de diphtérie conférant une protection contre la diphtérie, un antigène de la souche Neudörfl du virus de la TBE ou de la souche K23 du virus de la TBE conférant une protection contre l'encéphalite à tiques ainsi que des réactifs qui conviennent pour la préparation d'un vaccin combiné.

20. Kit selon la revendication 19, dans lequel lesdits antigènes sont des antigènes de Clostridium tétani, Corynebactérium diphtériae et de la souche Neudörfl du virus de la TBE ou de la souche K23 du virus de la TBE.

21. Kit selon les revendications 19 ou 20, où lesdits antigènes sont stockés séparément.

22. Kit selon la revendication 21, où les antigènes sont présents dans une seringue avec deux ou plusieurs chambres séparées.

23. Kit selon la revendication 21, où les antigènes sont présents en deux ou plusieurs flacons séparés.

24. Méthode de préparation d'un vaccin combiné selon l'une des revendications 1 à 16, comprenant les étapes de :
a) mélanger un toxoïde de tétanus conférant une protection contre le tétanus, un toxoïde de diphtérie conférant une protection contre la diphtérie et un antigène de la souche Neudörfl du virus de la TBE ou de la souche K23 du virus de la TBE conférant une protection contre l'encéphalite à tiques ; et
b) adsorber les antigènes mélangés à un adjuvant approprié.

25. Méthode de préparation d'un vaccin combiné selon l'une quelconque des revendications 1 à 16, comprenant les étapes de :
a) adsorber un toxoïde de tétanus conférant une protection contre le tétanus, un toxoïde de diphtérie conférant une protection contre la diphtérie et un antigène de la souche Neudörfl du virus de la TBE ou de la souche K23 du virus de la TBE conférant une protection contre l'encéphalite à tiques séparément les uns des autres à un adjuvant approprié ; et
b) mélanger les antigènes adsorbés les uns avec les autres.
